Europäisches Patentamt
European Patent Office
Office européen des brevets

(19)

(11) Publication number: **0 411 751 A1**

(12) **EUROPEAN PATENT APPLICATION**

(21) Application number: 90305978.0

(51) Int. Cl.[5]: C07K 5/02, A61K 37/64

(22) Date of filing: 31.05.90

The title of the invention has been amended (Guidelines for Examination in the EPO, A-III, 7.3).

(30) Priority: 06.06.89 GB 8912989
08.08.89 GB 8918073
08.12.89 GB 8927875

(43) Date of publication of application: 06.02.91 Bulletin 91/06

(84) Designated Contracting States: AT BE CH DE DK ES FR GB GR IT LI LU NL SE

(71) Applicant: Beecham Group p.l.c.
SB House Great West Road
Brentford Middlesex TW8 9BD(GB)

(72) Inventor: Smith, Stephen Allan, Beecham Pharmaceuticals
Coldharbour Road, The Pinnacles
Harlow, Essex CM19 5AD(GB)
Inventor: Ham, Peter, of Beecham Pharmaceuticals
Coldharbour Road, The Pinnacles
Harlow, Essex CM19 5AD(GB)

(74) Representative: Jones, Pauline et al
SmithKline Beecham, Corporate Patents,
Great Burgh, Yew Tree Bottom Road
Epsom, Surrey KT18 5XQ(GB)

(54) **Renin inhibitory peptides.**

(57) A compound of formula (I), or a pharmaceutically acceptable salt thereof:

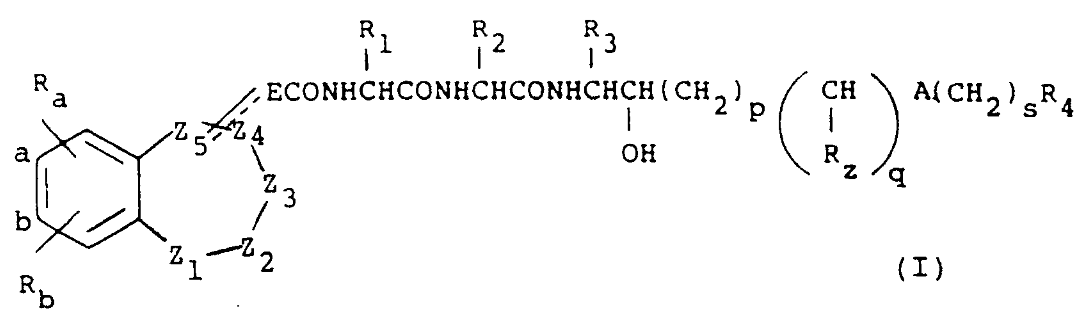

(I)

wherein
either $Z_1$ is absent and $Z_2$, $Z_3$, $Z_4$ and $Z_5$ and the carbon atoms to which $Z_2$ and $Z_5$ are attached, form a 6-membered non-aromatic heterocyclic ring; or
$Z_1$, $Z_2$, $Z_3$, $Z_4$, $Z_5$ and the carbon atoms to which $Z_1$ and $Z_5$ are attached, form a 7-membered non-aromatic heterocyclic ring;
E is absent or is $(CH_2)_n$ or $CH(CH_2)_{n-1}$ wherein n is 1 to 4;
A is -COHN-, -NHCO-, -COO-, $-S(O)_r-$ wherein r is 0, 1 or 2, or $-CH_2-$;
p is 0, 1 or 2;
s is 0, 1, 2, 3 or 4;
q is 0 or 1;
$R_z$ is hydrogen, $C_{1-6}$ alkyl or, when A is $-CH_2-$, hydroxy;

EP 0 411 751 A1

$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl, $C_{3-8}$ cycloalkyl or phenyl;
$R_4$ is $C_{1-6}$ alkyl, $C_{3-8}$ cycloalkyl, a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and
the dashed line represents an optional bond (when E is present);
which are renin inhibitors.

## NOVEL COMPOUNDS

The invention relates to novel compounds having pharmacological activity, to processes for their preparation, to pharmaceutical preparations containing them and to their use in medicine.

Renin is a natural enzyme, disorders in relation to which are implicated in many cases of hypertension. It is released into the blood from the kidney, and cleaves from a blood glycoprotein a decapeptide known as angiotensin-I. Circulating angiotensin-I is cleaved in plasma, and in lung, kidney and other tissues to an octapeptide, angiotensin-II, which raises blood pressure both directly by causing arteriolar constriction and indirectly by stimulating release of the sodium-retaining hormone aldosterone from the adrenal gland and thus causing a rise in extracellular fluid volume. The latter effect is caused by angiotensin-II itself or a heptapeptide cleavage product angiotensin-III.

Inhibitors of renin have been described as useful in the treatment of hypertension.

A group of compounds has now been discovered, which inhibit the enzyme renin and therefore have potential blood pressure lowering activity, useful in the treatment of hypertension. Compounds having an associated mechanism of action have also been described as possessing anti-retroviral activity and the present compounds may therefore be of potential use in the treatment of diseases caused by retroviruses including human immunodeficiency virus (HIV-1 and 2) and HTLV I and II. They may also be of potential use in the treatment of other cardiovascular disorders, such as congestive heart failure, and have beneficial effects on learning and memory and mood elevation activity, of potential use in the treatment of CNS disorders such as Alzheimer's disease and depression; they may also be of potential use in the treatment of glaucoma.

Accordingly, the present invention provides a compound of formula (I), or a pharmaceutically acceptable salt thereof:

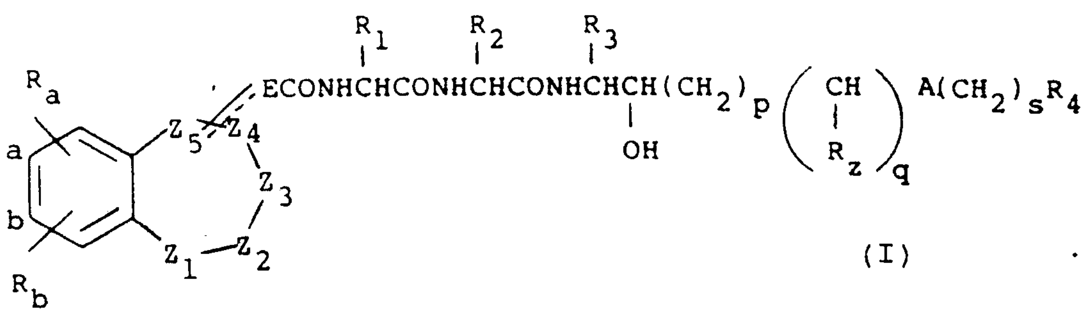

(I)

wherein

either $Z_1$ is absent and $Z_2$, $Z_3$, $Z_4$ and $Z_5$ and the carbon atoms to which $Z_2$ and $Z_5$ are attached, form a 6-membered non-aromatic heterocyclic ring; or

$Z_1$, $Z_2$, $Z_3$, $Z_4$, $Z_5$ and the carbon atoms to which $Z_1$ and $Z_5$ are attached, form a 7-membered non-aromatic heterocyclic ring;

E is absent or is $(CH_2)_n$ or $CH(CH_2)_{n-1}$ wherein n is 1 to 4;

A is -CONH-, -NHCO-, -COO-, $-S(O)_r-$ wherein r is 0, 1 or 2, or $-CH_2-$;

p is 0, 1 or 2;

s is 0, 1, 2, 3 or 4;

q is 0 or 1;

$R_z$ is hydrogen, $C_{1-6}$ alkyl or, when A is $-CH_2-$, hydroxy;

$R_a$ and $R_b$ are independently selected from hydrogen or a substituent;

$R_1$ is $CH_2R_9$ wherein $R_9$ is optionally substituted aryl or heteroaryl;

$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;

$R_3$ is $CH_2R_{12}$ wherein $R_{12}$ is $C_{1-6}$ alkyl, $C_{3-8}$ cycloalkyl or phenyl;

$R_4$ is $C_{1-6}$ alkyl, $C_{3-8}$ cycloalkyl, a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, $C_{1-6}$ alkylsulphonyl, carboxy, $C_{1-6}$ alkoxycarbonyl, benzyloxycarbonyl, aminocarbonyl or CH-

$(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and the dashed line represents an optional bond (when E is present).

When $Z_1$ is absent:

Values of $Z_2$, $Z_3$, $Z_4$ and $Z_5$ include wherein one of $Z_2$, $Z_3$, $Z_4$ and $Z_5$ is attached to E and is N, CH or C (wherein the optional exocyclic bond is present); another of $Z_2$, $Z_3$, $Z_4$ and $Z_5$ is O, S, SO, $SO_2$ or NR wherein R is hydrogen or $C_{1-6}$ alkyl; another of $Z_2$, $Z_3$, $Z_4$ and $Z_5$ is O, S, SO, $SO_2$, NR or $CH_2$; and the other of $Z_2$, $Z_3$, $Z_4$ and $Z_5$ is S, SO, $SO_2$, NR, $CH_2$ or C=O provided that, one of $Z_2$ and $Z_3$, one of $Z_3$ and $Z_4$, or one of $Z_4$ and $Z_5$ is $CH_2$, CH or C when the other is S or O, or is $CH_2$, CH, C, NR or N when the other is SO or $SO_2$; or $Z_3/Z_4$ is SO, $SO_2$ or C=O when one of $Z_2/Z_3$ and $Z_4/Z_5$ is N or and the other is O, S, N or NR; or $Z_2$-$Z_3$-$Z_4$-$Z_5$ is -NRCOCON< or >NCOCONR-; or $Z_2/Z_5$ is CO, $Z_3/Z_4$ is O and $Z_4$,$Z_5$/$Z_2$,$Z_3$ are CH and $CH_2$.

i.e. the following are excluded in $Z_2$ to $Z_5$:-

i) adjacent same heteroatoms (O, S, N) except N-N; but no N-N-N;

iii) $SO_x$-$SO_y$ or O-$S_y$ (x and y are 0-2);

iv) O-CO, S-C-O, S-C-S;

v) CO-SO or CO-$SO_2$;

vi) two heteroatoms (O, S, N) separated by a Z when CO unless one of them is N;

vii) more than two of $Z_2$ to $Z_5$ is CO.

Examples of $Z_2$, $Z_3$, $Z_4$ and $Z_5$ therefore include

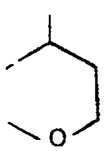 , 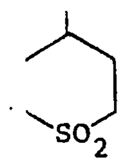 , 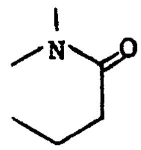 ,

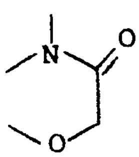 , 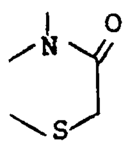 , 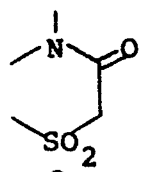 ,

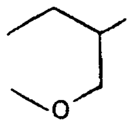 , 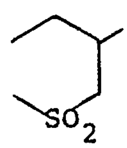 , 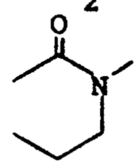 ,

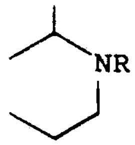 , 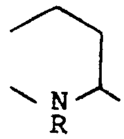 , 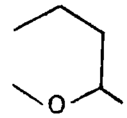 ,

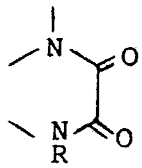 , 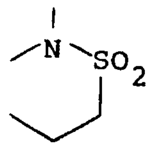 , 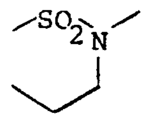 ,

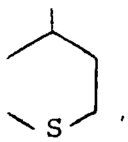 , 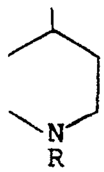 , 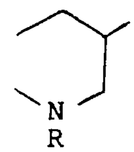 ,

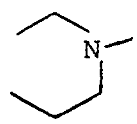

Preferably $Z_2$ is $SO_2$ or O, $Z_3$ is $CH_2$, $Z_4$ is $CH_2$, $Z_5$ is CH and E is attached at $Z_5$; or $Z_2$ is $SO_2$, $Z_3$ is CH, $Z_4$ is CO, and $Z_5$ is N and E is attached at $Z_5$.

<u>When $Z_1$ is present:</u>

Heterocyclic ring heteroatoms for $Z_1$ to $Z_5$ are selected from oxygen, sulphur, SO, $SO_2$, nitrogen or N-alkyl. The ring may include unsaturation (C = C) or a carbon attached to exocyclic oxo (C = O).

Examples of $Z_1$, $Z_2$, $Z_3$, $Z_4$ and $Z_5$ then include

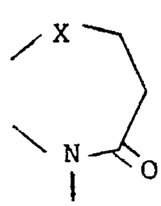 , 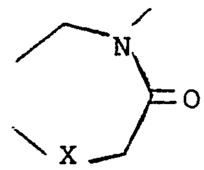 , 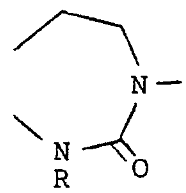 , 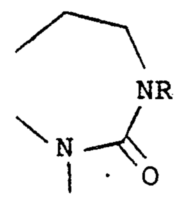

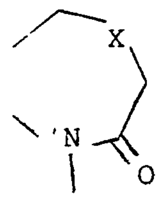 , 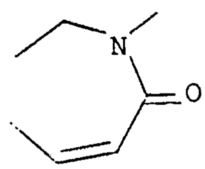 , 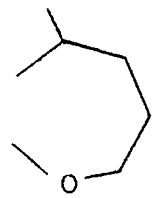

wherein X is O, S, SO, $SO_2$, NH, N-$C_{1-6}$ alkyl or $CH_2$.

Preferably E is attached at a nitrogen atom.

Suitable examples of $R_a$ and $R_b$ include a moiety selected from halogen, $C_{1-6}$ alkyl, $C_{1-6}$ alkoxy, nitro, cyano, SH, $SO_3H$, CHO, $C_{1-6}$ alkyl substituted by hydroxy, $C_{1-6}$ alkanoyloxy, optionally substituted amino or by $C_{1-7}$ alkanoylamino, a group $NR_5R_6$, $SO_2NR_5R_6$, $CO_2R_5$, $NHCONR_5R_6$ or $NHCOR_5$ wherein $R_5$ is hydrogen, $C_{1-6}$ alkyl or optionally substituted benzyl and $R_6$ is hydrogen or $C_{1-6}$ alkyl, a group $S(O)_mR_7$ wherein m is 0, 1 or 2 and $R_7$ is hydrogen or $C_{1-6}$ alkyl optionally substituted by amino, acylamino, protected amino, or $CO_2H$ or a pharmaceutically acceptable ester (such as a $C_{1-6}$ alkyl ester) thereof; or a group $CH=NR_8$ wherein $R_8$ is $C_{1-6}$ alkyl optionally substituted by amino or hydroxy. $R_a$ or $R_b$ may also be a mercapto substituent such that the resulting compound is a disulphide.

Suitable examples of substituents in optionally substituted amino groups in $R_a/R_b$ then include one or two groups independently selected from $C_{1-6}$ alkyl or $C_{1-6}$ alkyl substituted by carboxy, $C_{1-6}$ alkoxycarbonyl, hydroxy or amino group.

Alternatively, $NR_5R_6$ or other substituted amino groups in $R_a/R_b$ may be pyrrolidinyl, piperidinyl, morpholinyl or piperazinyl optionally N-substituted by $C_{1-6}$ alkyl.

Suitable acyl groups in $R_7$ when $C_{1-6}$ alkyl substituted by acylamino, include $C_{1-7}$ alkanoyl, or optionally substituted benzoyl. Suitable protecting groups in protected amino include benzyloxycarbonyl and t-butyloxycarbonyl.

Preferably one of $R_a$ and $R_b$ is hydrogen and the other is $CH_2NH_2$, $CO_2H$, $CH_2NHCH_2CO_2H$ or $SO_2CH_2CO_2H$, attached at carbon atom a or b depicted in formula (I).

In regard to values of A and p and q, reference is hereby made to the following literature:-

1. J. Med. Chem. 1988, 31 , 1918
(p = 1, q = 0, A = -CONH-)
2. J. Med. Chem. 1987, 30 , 1224
(p = 2, q = 0, A = -NHCO-)
3. J. Med. Chem. 1987, 31 , 701
(p = 0, q = 0, A = -COO-)
4. J. Med. Chem. 1987, 31 , 1839
(p = 1, q = 1, A = -CONH-)
5. J. Med. Chem. 1987, 30 , 1729 and refs. therein
(p = 2, q = 1, A = $S(O)_r$)
6. WO 88/05050
(p = 0, q = 1, A = $CH_2$, $R_z$ = OH)
7. EP-A-172346
(p = 0, q = 1, A = $CH_2$, $R_z$ = H)

Suitable values of $R_9$ and $R_{11}$ when aryl include phenyl or naphthyl and when heteroaryl, include a 5- or 6-membered monocyclic or 9- or 10- membered bicyclic of which 5- or 6- membered monocyclic

heteroaryl is preferred. In addition, 5- or 6-membered monocyclic or 9- or 10-membered bicyclic heteroaryl preferably contains one, two or three heteroatoms which are selected from the class of oxygen, nitrogen and sulphur and which, in the case of there being more than one heteroatom, are the same or different. Examples of 5-or 6-membered monocyclic heteroaryl containing one, two or three heteroatoms which are selected from the class of oxygen, nitrogen and sulphur include furyl, thienyl, pyrryl, oxazolyl, thiazolyl, imidazolyl and thiadiazolyl, and pyridyl, pyridazyl, pyrimidyl, pyrazolyl and triazolyl. Preferred examples of such groups include furanyl, thienyl, pyrryl and pyridyl, in particular 2- and 3-furanyl, 2- and 3-pyrryl, 2- and 3-thienyl, and 2-, 3- and 4-pyridyl. Examples of 9- or 10-membered bicyclic heteroaryl containing one, two or three heteroatoms which are selected from the class of oxygen, nitrogen and sulphur include benzofuranyl, benzothienyl, indolyl and indazolyl, quinolyl and isoquinolyl, and quinazolyl. Examples of such groups include 2- and 3-benzofuranyl, 2- and 3-benzothienyl, and 2- and 3-indolyl, and 2- and 3-quinolyl and, for $R_{11}$, 4-benzimidazolyl.

Suitable examples of groups or atoms for optional substitution of $R_5$ when benzyl and $R_9$ and $R_{11}$ include one, two or three substituents independently selected from $C_{1-4}$ alkyl, $C_{1-4}$ alkoxy, halo (such as fluoro, chloro, bromo), hydroxy, nitro, and cyano.

Preferred examples of $R_9$ include phenyl and naphthyl, and preferred examples of $R_{11}$ when aryl or heteroaryl include phenyl, imidazol-4-yl and -2-yl, pyrazol-1-yl and 4-methylpyrazol-1-yl.

Preferably the amino acid residue containing $R_2$ is Leu, β-pyrazolylalanine or His.

Abbreviations which may be used herein are as follows:

| Amino Acid Residue | Abbreviations |
|---|---|
| histidine | His |
| isoleucine | Ile |
| leucine | Leu |
| 1-naphthylalanine | NAla |
| norleucine | Nle |
| phenylalanine | Phe |

The α-amino acid components are in the (S)-configuration (or L-form).

In a particular aspect, the present invention provides a compound of formula (IA) or a pharmaceutically acceptable salt thereof:

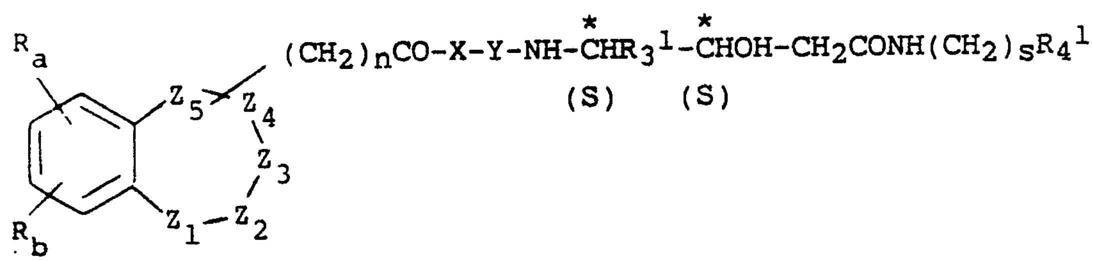

(IA)

wherein

X is Phe;

Y is Leu or His;

$R_3{}^1$ is cyclohexylmethyl;

$R_4{}^1$ is $C_{4-5}$ alkyl and s is 0 or s is 2 to 4 and $R_4{}^1$ is carboxy or a saturated or unsaturated heterocyclic ring; and

the remaining variables are as defined in formula (I).

Suitable values for alkyl groups in $R_a$, $R_b$, $R_z$, R and $R_2$ to $R_{12}$ in formulae (I) and (IA) include methyl, ethyl, n - and iso -propyl, n -, sec -, iso - and tert -butyl, n -, iso -, sec -, tert - and neo -pentyl. $C_{3-8}$ cycloalkyl groups are cyclopropyl, cyclobutyl, cyclopentyl, cyclohexyl, cycloheptyl and cyclooctyl.

Suitable values for $R_a$ and $R_b$ halo include fluoro, chloro, bromo and iodo, preferably chloro or bromo.

Preferably $R_4$ or $R_4^1$ when alkyl is iso -butyl.

Suitable examples of $R_4$ when monocyclic heteroaryl include those listed for $R_9$ and $R_{11}$, preferably 3-pyridyl or an N-oxide thereof, or 1-imidazolyl.

When $R_4$ is a saturated heterocyclic ring, suitable examples include piperidinyl, pyrrolidinyl, piperazinyl and morpholinyl; optionally N-substituted by $C_{1-6}$ alkyl group(s), or as an N-oxide thereof.

The right hand sequence in formula (I) and (IA) is preferably 4(S)-amino-5-cyclohexyl-3(S)-hydroxy-pentanoic acid (ACHPA), from the amino acid containing $R_3$ or $R_3^1$, to $NH(CH_2)_sR_4$.

Preferably n is 1, 2 or 3.

Preferably E is attached at $Z_5$.

Pharmaceutically acceptable salts include acid addition salts which may be, for example, salts with inorganic acids such, for example, as hydrochloric acid, hydrobromic acid, orthophosphoric acid or sulphuric acid, or organic acids such, for example, as methanesulphonic acid, toluenesulphonic acid, acetic acid, trifluoroacetic acid, propionic acid, lactic acid, citric acid, tartaric acid, fumaric acid, malic acid, succinic acid, salicylic acid or acetylsalicylic acid.

Pharmaceutically acceptable salts may also include alkali metal salts, for example sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine.

It will be appreciated that the compounds of the invention may exist as solvates, such as hydrates, and these are included whenever, a compound of formula (I) or a salt thereof, is herein referred to.

The compounds of formula (I) and (IA) wherein E is attached to $Z_1/Z_2/Z_3/Z_4/Z_5$ when CH, have at least one asymmetric centre in addition to those attached to $R_1$ and $R_2$ (in X and Y) and $R_3$ and those indicated in formula (IA), and are therefore capable of existing in more than one stereoisomeric form. The invention extends to each of these forms and to mixtures thereof. Preferably the configuration at the carbon atoms bearing $R_1$, $R_2$ and $R_3$ is the (S)-configuration.

It will be appreciated that, when the optional bond depicted in formula (I) is present, the compounds are capable of existing in E and Z ( trans and cis ) forms. The invention extends to each of these forms and to mixtures thereof.

The compounds of the invention are preferably in pharmaceutically acceptable form. By pharmaceutically acceptable form is meant, inter alia, of a pharmaceutically acceptable level of purity excluding normal pharmaceutical additives such as diluents and carriers, and including no material considered toxic at normal dosage levels. A pharmaceutically acceptable level of purity will generally be at least 50% excluding normal pharmaceutical additives, preferably 75%, more preferably 90% and still more preferably 95%.

A compound of the invention may be prepared by those methods known in the art for the synthesis of compounds of analogous structure, such as peptides, and in this regard reference is made, by way of illustration only, to the literature reference: S.R. Pettit, " Synthetic Peptides " (Elsevier Scientific Publishing Co. 1976).

The present invention also provides a compound of the present invention which has been prepared synthetically.

A compound of the present invention may, for example, be formed by the sequential coupling of appropriate amino acids with the acid of formula (II):

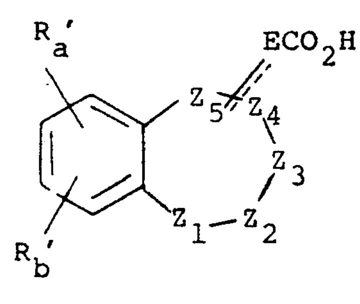

(II)

wherein $R_a'$ and $R_b'$ are $R_a$ and $R_b$ respectively or groups or atoms convertible thereto; or by the initial preparation and subsequent coupling of peptide subunits with the acid of formula (II), the subunits themselves prepared in stepwise manner; in either case classical solution chemistry methods analogous to those used in peptide synthesis, may be employed.

The coupling reactions may be effected by, for example, activating the reacting carboxyl group of the

acid of formula (II) or amino acid, and reacting this with the amino group of the substrate unit. Details of suitable, optional activating and protecting (masking) groups and of suitable reaction conditions (for the coupling reactions and for the introduction and removal of protecting groups) giving, preferably, the minimum of racemisation, may be found in the above-referenced literature.

It will be appreciated that, when A is other than -CONH- or -COO-, the C-terminus group is other than an amino acid, in which case the coupling is carried out with an appropriate other precursor, as described in the aforementioned literature references 2., 5., 6. and 7.

Accordingly, the present invention further provides a process for the preparation of a compound of formula (I) or a pharmaceutically acceptable salt thereof which comprises reacting a reagent of the formula (III):

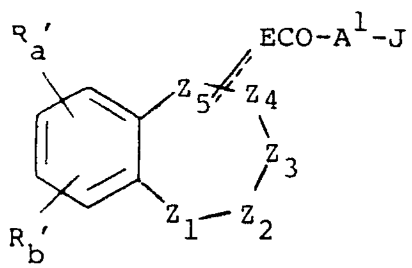

(III)

wherein $R_a'$ and $R_b'$ are $R_a$ and $R_b$ respectively or groups or atoms convertible thereto; $A^1$ is absent or represents an appropriate amino acid or dipeptide unit; J is OH or a leaving group; and the remaining variables are as hereinbefore defined; with a reagent of the formula (IV):

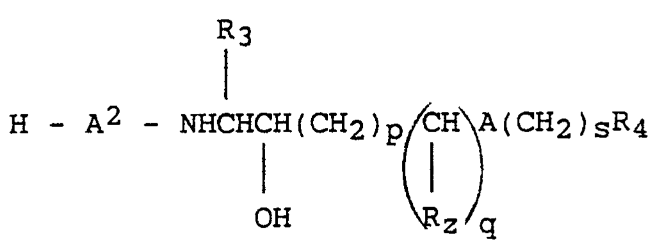

(IV)

wherein

$A^2$ is absent or represents an appropriate amino acid or dipeptide unit, such that $A^1$ + $A^2$ is $-NHCHR_1CONHCHR_2CO-$; and the remaining variables are as hereinbefore defined; and thereafter, if desired or necessary deprotecting (within $A^1$ or $A^2$) of the products, and/or converting $Z_1$, $Z_2$, $Z_3$, $Z_4$ or $Z_5$ to other $Z_1$, $Z_2$, $Z_3$, $Z_4$ or $Z_5$, $R_a'$ /$R_b'$ to $R_a$ and/or $R_b$ and/or forming a pharmaceutically acceptable salt thereof.

Suitable examples of J when a leaving group include halo, such as chloro or bromo, and other suitable groups which are displaceable by an amino nucleophile, such as $C_{1-6}$ alkoxycarbonyloxy.

It is generally preferred, especially when $A^1$ is not absent, however, that J is OH, and a suitable coupling reagent or dehydrating catalyst is used to effect the reaction, such as N,N-dicyclohexylcarbodiimide and those described in the Descriptions and Examples hereinafter.

Deprotection of $A^1$ and/or $A^2$ takes place conventionally, in accordance with the particular protecting group(s) employed.

Pharmaceutically acceptable salts may be formed conventionally.

$Z_1$, $Z_2$, $Z_3$, $Z_4$ or $Z_5$ when S or SO may be converted to SO or $SO_2$ respectively (or S to $SO_2$) by conventional oxidation methods, such as those described hereinafter for conversion (vi) for $R_a/R_b$.

It will be apparent that compounds of the formula (I) containing an $R_a'$ $R_b'$ group which is other than $R_a/R_b$ and which is convertible to $R_a/R_b$ group, are useful novel intermediates. A number of such conversions is possible, not only for the final product compounds of formula (1) when $R_a'/R_b'$ are other than $R_a/R_b$, but also within $R_a/R_b$, and also for their intermediates as follows:

(i) a hydrogen substituent is convertible to a nitro substituent by nitration;

(ii) a nitro substituent is convertible to an amino substituent by reduction;
(iii) a $C_{1-7}$ acylamino substituent is convertible to an amino substituent by deacylation;
(iv) an amino substituent is convertible to a $C_{1-7}$ acylamino substituent by acylation with a carboxylic acid derivative;
(v) a hydrogen substituent is convertible to a halogen substituent by halogenation;
(vi) an $C_{1-6}$ alkylthio or a $C_{1-6}$ alkylsulphinyl substituent is convertible to a $C_{1-6}$ alkylsulphinyl or $C_{1-6}$ alkylsulphonyl substituent respectively, by oxidation;
(vii) an amino, aminosulphonyl, or $NHCONH_2$ substituent is convertible to a corresponding substituent which is substituted by one or two alkyl groups, by N-alkylation;
(viii) an amino substituent is convertible to a group $NHCONH_2$, by reaction with potassium cyanate and acid;
(ix) a hydrogen substituent is convertible to an aminosulphonyl substituent by treatment with $ClSO_3H$ followed by $HNR_5R_6$;
(x) a cyano substituent may be converted to an aminomethyl substituent by reduction;
(xi) a bromo substituent may be converted to a cyano substituent by reaction with copper(I) cyanide.

Conversions (i) to (xi) are only exemplary and are not exhaustive of the possibilities. It will be appreciated that it is often desirable to carry out these conversions at an earlier stage, in the intermediate acid of formula (II), especially in regard to conversion (iii).

In regard to (i), nitration is carried out in accordance with known procedures.

In regard to (ii), the reduction is carried out with a suitable reducing agent, such as that suitable for reducing nitroanisole to aminoanisole.

In regard to (iii), deacylation is carried out by treatment with a base, such as an alkali metal hydroxide.

In regard to (iv), the acylation is carried out with an acylating agent, such as the corresponding acid or acid chloride. Formylation is carried out with the free acid.

In regard to (v), halogenation is carried out with conventional halogenating agents.

In regard to (vi), oxidation is carried out at below ambient temperatures in a non-aqueous solvent, such as a chlorinated hydrocarbon, in the presence of an organic peracid, such as 3-chloroperoxybenzoic acid, or in water in the presence of a soluble strong inorganic oxidant, such as an alkali metal permanganate or with aqueous hydrogen peroxide.

In regard to (vii), alkylation is carried out with a corresponding alkylating agent such as the chloride or bromide under conventional conditions.

In regard to (viii), conversion to a ureido derivative is carried out by reaction with potassium cyanate in acidic methanol, at ambient temperature.

In regard to (ix), the mixing with $ClSO_3H$ takes place at low temperatures around 0°C and allowed to warm to ambient temperature. The subsequent reaction with the amine takes place at ambient temperature, in a solvent such as ethanol.

In regard to (x), the reduction takes place by reaction with sodium borohydride/cobalt chloride; or platinum oxide/hydrogen.

In regard to (xi), the reaction takes place under conventional conditions.

Compounds of the general formulae (II) and (III) may themselves be prepared by standard techniques analogous to those described above.

The acids of formula (II) are either known compounds or are prepared by analogous methods to these and for structurally similar known compounds.

The preparation of the amino acid of formula (V):

$$H_2N-\overset{*}{C}HR_3{}^1-\overset{*}{C}HOH-CH_2CO_2H \qquad (V)$$

(S) (S)

is described in J. Med. Chem 1985, 28 , 1779-1790.

As regards the appropriate corresponding intermediates to those of formula (V), including those wherein A is other than -CONH-, reference is hereby made to the literature references 2. to 7. listed hereinbefore.

It will be appreciated that protected forms of compounds of the present invention are novel intermediates and form an aspect of the invention.

A particularly suitable method of preparation of the compounds of the present invention are as described in the Description and Examples hereinafter. The couplings may be carried out sequentially

beginning by coupling the acid of formula (II) with, for example, phenylalanine or 1-naphthylalanine, followed by coupling with Y, for example, leucine or histidine, and finally, coupling with the amino acid of formula (V). In a preferred aspect, however, either the acid of formula (II) is coupled with a tripeptide unit formed between the amino acid of formula (V); leucine, histidine or other $R_2$ containing amino acid; and phenylalanine or naphthylalanine; or alternatively the acid of formula (II) is coupled with phenylalanine or naphthylalanine and this is coupled with a dipeptide unit formed between the $R_2$ containing amino acid and the amino acid of formula (V).

As mentioned previously the compounds of the invention have been found to be renin inhibitors, and therefore they are of potential use in the treatment of hypertension. They may also be of potential use in the other diseases and disorders hereinbefore referred to.

The present invention also provides a pharmaceutical composition which comprises a compound of formula (I) or a pharmaceutically acceptable salt thereof, and a pharmaceutically acceptable carrier. In particular, the present invention provides an anti-hypertensive pharmaceutical composition which comprises an anti-hypertensive effective amount of a compound of formula (I) or a pharmaceutically acceptable salt thereof, and a pharmaceutically acceptable carrier.

The compositions are preferably adapted for oral administration. However, they may be adapted for other modes of administration, for example parenteral administration for patients suffering from heart failure. Other alternative modes of administration include sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration.

The present invention further provides a method of prophylaxis or treatment of hypertension in mammals including man, which comprises administering to the suffering mammal an anti-hypertensively effective amount of a compound of formula (I) or a pharmaceutically acceptable salt thereof.

An effective amount will depend on the relative efficacy of the compounds of the present invention, the severity of the hypertension being treated and the weight of the sufferer. However, a unit dose form of a

composition of the invention may contain from 0.1 to 500 mg of a compound of the invention and more usually from 1 to 100 mg, for example 2 to 50 mg such as 2, 3, 4, 5, 10, 20 or 30mg. Such compositions may be administered from 1 to 6 times a day, more usually from 2 to 4 times a day, in a manner such that the daily dose is from 1 to 1000mg for a 70 kg human adult and more particularly from 5 to 500 mg.

No toxicological effects are indicated at the aforementioned dosage ranges.

The present invention further provides a compound of formula (I) or a pharmaceutically acceptable salt thereof for use in the treatment or prophylaxis of hypertension.

The following Description relates to the preparation of intermediates and the following Examples relate to the preparation of compounds of formula (I).

The following Tabulations show the structures prepared. The following notations may be employed:-

| | |
|---|---|
| 4(S)-amino-5-cyclohexyl-3(S)-hydroxypentanoic acid isobutylamide | ACHPAA |
| 4(S)-amino-5-cyclohexyl-3(S)-hydroxypentanoic acid | ACHPA |
| 4(S)-amino-5-cyclohexyl-3(S)-hydroxypentanoic acid 3-(1-imidazolyl)propylamide | ACHPA-API |
| Trifluoroacetate Salt | TFA |
| Benzyloxycarbonyl | CBZ |
| tert -Butyloxycarbonyl | BOC |
| Point of attachment of E | * |

## Tabulated Structures : 1

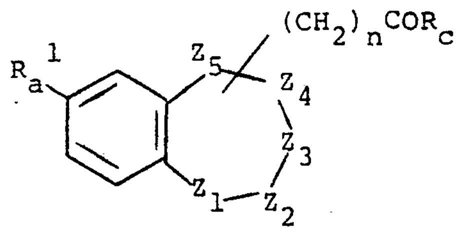

| Desc. No. | $R_a^1$ | $Z_1$ | $Z_2$ | $Z_3$ | $Z_4$ | $Z_5$ | n | $R_c$ |
|---|---|---|---|---|---|---|---|---|
| D1(b) | H | - | O | $CH_2$ | $CH_2$ | CH* | 1 | OEt |
| D1(c) | H | - | O | $CH_2$ | $CH_2$ | CH* | 1 | OH |
| D2(c) | $H_3C$ | - | O | $CH_2$ | CO | N* | 1 | OMe |
| D2(d) | $H_3C$ | - | O | $CH_2$ | CO | N* | 1 | OH |
| D5(d) | H | - | $SO_2$ | $CH_2$ | $CH_2$ | CH* | 1 | OH |
| D6(c) | NC | - | O | $CH_2$ | CO | N* | 2 | OMe |
| D6(d) | NC | - | O | $CH_2$ | CO | N* | 2 | OH |
| D7(a) | H | - | S | $CH_2$ | CO | N* | 2 | OMe |
| D7(b) | H | - | S | $CH_2$ | CO | N* | 2 | OH |
| D8(a) | H | - | $SO_2$ | $CH_2$ | CO | N* | 2 | OMe |
| D8(b) | H | - | $SO_2$ | $CH_2$ | CO | N* | 2 | OH |
| D10(b) | BOC-$HNH_2C$ | - | O | $CH_2$ | CO | N* | 2 | OMe |
| D10(c) | BOC-$HNH_2C$ | - | O | $CH_2$ | CO | N* | 2 | OH |
| D11 | H | - | $CH_2$ | $CH_2$ | N* | $CH_2$ | 2 | OH |
| D13(f) | $PhCH_2O_2C$ | - | O | $CH_2$ | CO | N* | 2 | OH |
| D14(f) | $PhCH_2O_2CH_2C$ | - | O | $CH_2$ | CO | N* | 2 | OH |
| D15(a) | H | S | $CH_2$ | $CH_2$ | CO | N* | 2 | $O^tBu$ |
| D15(b) | H | S | $CH_2$ | $CH_2$ | CO | N* | 2 | OH |
| D16(a) | H | $SO_2$ | $CH_2$ | $CH_2$ | CO | N* | 2 | $O^tBu$ |
| D16(b) | H | $SO_2$ | $CH_2$ | $CH_2$ | CO | N* | 2 | OH |
| D17(c) | NC | - | S | $CH_2$ | CO | N* | 2 | OMe |
| D17(d) | BOC-$HNH_2C$ | - | S | $CH_2$ | CO | N* | 2 | OMe |
| D17(e) | BOC-$HNH_2C$ | - | S | $CH_2$ | CO | N* | 2 | OH |

## Tabulated Structures : 2

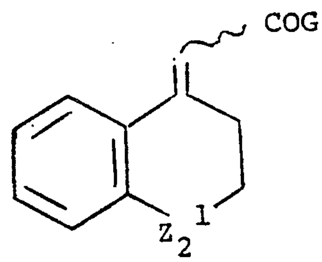

| Desc./Ex.No. | $Z_2^1$ | G | Salt/solvate |
|---|---|---|---|
| D1(a) | O | OEt | |
| D5(b) | $SO_2$ | OEt | |
| D5(c) | $SO_2$ | OH | |
| E3 | $SO_2$ | PheLeuACHPAA | $0.5H_2O$ |

## Tabulated Structures : 3

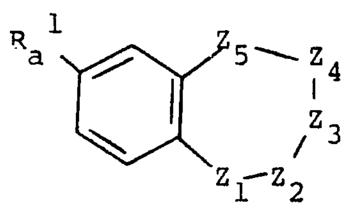

| Desc. No. | $R_a^1$ | $Z_1$ | $Z_2$ | $Z_3$ | $Z_4$ | $Z_5$ |
|---|---|---|---|---|---|---|
| D2(b) | $H_3C$ | - | O | $CH_2$ | CO | NH |
| D5(a) | H | - | $SO_2$ | $CH_2$ | $CH_2$ | CO |
| D6(b) | NC | - | O | $CH_2$ | CO | NH |
| D10(a) | BOC-$HNH_2C$ | - | O | $CH_2$ | CO | NH |
| D13(c) | $^tBuO_2CCH_2O_2C$ | - | O | $CH_2$ | CO | NH |
| D13(d) | $HO_2C$ | - | O | $CH_2$ | CO | NH |
| D13(e) | $PhCH_2O_2C$ | - | O | $CH_2$ | CO | NH |
| D14(c) | $^tBuO_2CCH_2O_2CH_2C$ | - | O | $CH_2$ | CO | NH |
| D14(d) | $HO_2CH_2C$ | - | O | $CH_2$ | CO | NH |
| D14(e) | $PhCH_2O_2CH_2C$ | - | O | $CH_2$ | CO | NH |
| D17(b) | NC | - | S | $CH_2$ | CO | NH |

| Tabulated Structures : 4 | | | |
|---|---|---|---|
| $R_d$-ACHPA-$R_e$ | | | |
| Desc. No. | $R_d$ | $R_e$ | Salt/Solvate |
| D3(a) | BOC-Phe-Leu | $NH^iBu$ | |
| D3(b) | Phe-Leu | $NH^iBu$ | TFA |
| D4(a) | CBZ-Phe-Leu | $NH^iBu$ | |
| D4(b) | Phe-Leu | $NH^iBu$ | $CH_3CO_2H$ |
| D4(c) | Phe-Leu | $NH^iBu$ | |
| D9(a) | BOC | API | |
| D9(b) | CBZ-Phe-Leu | API | $0.5H_2O$ |
| D9(c) | Phe-Leu | API | |
| D12(a) | BOC-Phe-Leu | API | |
| D12(b) | Phe-Leu | API | 2TFA |

## Tabulated Structures : 5

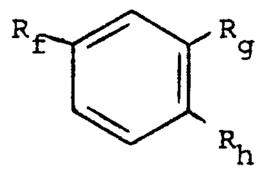

| Desc. No. | $R_f$ | $R_g$ | $R_h$ |
|---|---|---|---|
| D2(a) | $H_3C$ | $NHCOCH_2Cl$ | OH |
| D6(a) | NC | $NO_2$ | $OCH_2CO_2{}^tBu$ |
| D13(a) | $^tBuO_2CCH_2O_2C$ | $NO_2$ | $OCH_2CO_2{}^tBu$ |
| D13(b) | $^tBuO_2CCH_2O_2C$ | $NH_2$ | $OCH_2CO_2{}^tBu$ |
| D14(a) | $^tBuO_2CH_2CO_2CH_2C$ | $NO_2$ | $OCH_2CO_2{}^tBu$ |
| D14(b) | $^tBuO_2CH_2CO_2CH_2C$ | $NH_2$ | $OCH_2CO_2{}^tBu$ |
| D17(a) | NC | $NO_2$ | $SCH_2CO_2Et$ |

Tabulated Structures : 6

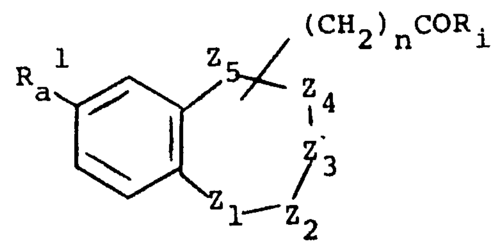

| Ex. No. | $R_a^1$ | $Z_1$ | $Z_2$ | $Z_3$ | $Z_4$ | $Z_5$ | n | $R_i$ | Salt/solvate |
|---|---|---|---|---|---|---|---|---|---|
| E1 | H | - | O | $CH_2$ | $CH_2$ | CH* | 1 | PheLeuACHPAA | $0.5H_2O$ |
| E2 | $H_3C$ | - | O | $CH_2$ | CO | N* | 1 | PheLeuACHPAA | $H_2O$ |
| E4 | H | - | $SO_2$ | $CH_2$ | $CH_2$ | CH* | 1 | PheLeuACHPAA | $0.5H_2O$ |
| E5 | NC | - | O | $CH_2$ | CO | N* | 2 | PheLeuACHPAA | $0.5H_2O$ |
| E6 | H | - | S | $CH_2$ | CO | N* | 2 | PheLeuACHPAA | $0.5H_2O$ |
| E7 | H | - | $SO_2$ | $CH_2$ | CO | N* | 2 | PheLeuACHPAA | $0.5H_2O$ |
| E8a | H | - | $SO_2$ | $CH_2$ | CO | N* | 2 | PheLeuACHPA-API | $1.5H_2O$ |
| E8b | H | - | $SO_2$ | $CH_2$ | CO | N* | 2 | PheLeuACHPA-API | HCl |
| E9 | H | - | NH | CO | CO | N* | 1 | PheLeuACHPAA | $H_2O$ |
| E10 | BOC-$HNH_2C$ | - | O | $CH_2$ | CO | N* | 2 | PheLeuACHPA-API | $0.5CHCl_3$ |

Tabulated Structures : 6 (Contd.)

| Ex. No. | $R_a^1$ | $Z_1$ | $Z_2$ | $Z_3$ | $Z_4$ | $Z_5$ | n | $R_i$ | Salt/solvate |
|---|---|---|---|---|---|---|---|---|---|
| E11 | $H_2NH_2C$ | - | O | $CH_2$ | CO | N* | 2 | PheLeuACHPA-API | 3TFA |
| E12 | H | - | $CH_2$ | $CH_2$ | N* | $CH_2$ | 2 | PheLeuACHPA-API | |
| E13a | H | - | NH | CO | CH* | S | 1 | PheLeuACHPA-API | |
| E13b | H | - | NH | CO | CH* | S | 1 | PheLeuACHPA-API | $2H_2O.HCl$ |
| E14 | $PhCH_2O_2C$ | - | O | $CH_2$ | CO | N* | 2 | PheLeuACHPA-API | |
| E15a | $HO_2C$ | - | O | $CH_2$ | CO | N* | 2 | PheLeuACHPA-API | |
| E15b | $HO_2C$ | - | O | $CH_2$ | CO | N* | 2 | PheLeuACHPA-API | $2.5H_2O.HCl$ |
| E16 | $PhCH_2O_2CH_2C$ | - | O | $CH_2$ | CO | N* | 2 | PheLeuACHPA-API | |
| E17a | $HO_2CH_2C$ | - | O | $CH_2$ | CO | N* | 2 | PheLeuACHPA-API | |
| E17b | $HO_2CH_2C$ | - | O | $CH_2$ | CO | N* | 2 | PheLeuACHPA-API | HCl |
| E18 | H | S | $CH_2$ | $CH_2$ | CO | N* | 2 | PheLeuACHPA-API | $0.5CHCl_3$ |
| E19a | H | $SO_2$ | $CH_2$ | $CH_2$ | CO | N* | 2 | PheLeuACHPA-API | $2.5H_2O$ |
| E19b | H | $SO_2$ | $CH_2$ | $CH_2$ | CO | N* | 2 | PheLeuACHPA-API | $1.5H_2O.HCl$ |
| E20 | BOC-$HNH_2C$ | - | S | $CH_2$ | CO | N* | 2 | PheLeuACHPA-API | |
| E21 | $H_2NH_2C$ | - | S | $CH_2$ | CO | N* | 2 | PheLeuACHPA-API | 2TFA |
| E22 | $H_2NH_2C$ | - | $SO_2$ | $CH_2$ | CO | N* | 2 | PheLeuACHPA-API | 2TFA |

Description 1

(a) Ethyl chromanylidene-4-acetate

Sodium hydride (80% in mineral oil, 1.11g) was stirred under nitrogen in dry dimethoxyethane (50ml) as triethyl phosphonoacetate (6.7ml) was added dropwise over 25 min. After stirring for a further 20 min, 4-chromanone (4.58g) was added dropwise in dry dimethoxyethane (15ml). The solution was stirred for 16h and poured into water. The product was extracted into ether, dried ($Na_2SO_4$), and evaporated in vacuo . Chromatography on silica gel using 20% ethyl acetate in petroleum ether (b.p.60-80°C) gave the title compound (1.83g) as a colourless oil. NMR showed this to be a ca 2:1 mixture of isomers.
NMR (δ)($CDCl_3$): 1.3 (3H,m), 2.65 (dt)(minor) and 3.4 (dt)(major) (2H), 4.1-4.4 (4H,m), 5.7(s) (minor) and 6.35(s)(major)(1H), 6.8-7.0 (2H,m), 7.25 (1H,m), 7.6 (dd)(major) and 7.8(dd)(minor)(1H).

(b) Ethyl chromanyl-4-acetate

Ethyl chromanylidene-4-acetate (1.82g) was hydrogenated over 10% palladium on charcoal (0.52g) in ethanol (50ml) for 1h. Catalyst was filtered off, and the filtrate was evaporated in vacuo , giving the title compound (1.71g) as a colourless oil.
NMR (δ) ($CDCl_3$): 1.3 (3H,t), 1.85 (1H,m), 2.15 (1H, m), 2.5 (1H) and 2.8 (1H) (ABX), 3.35 (1H, m), 4.2 (2H, q), 6.75-6.9 (2H, m), 7.1 (2H, m).

(c) Chromanyl-4-acetic acid

Ethyl chromanyl-4-acetate (1.71g) and 10% aqueous sodium hydroxide (4.3ml) were dissolved in ethanol (20ml), and left to stand for 2h. The mixture was diluted with water, washed with chloroform, acidified (5M hydrochloric acid), and extracted with ethyl acetate. The extract was dried ($Na_2SO_4$) and evaporated in vacuo , giving the title compound (1.33g) as a white solid.
NMR (δ) ($CDCl_3$): 1.9 (1H, m), 2.2 (1H, m), 2.6 (1H) and 2.9 (1H) (ABX), 3.4 (1H, m), 4.2 (2H, m), 6.75-6.9 (2H, m), 7.1 (2H, m).

Description 2

(a) 2-(Chloroacetylamino)-4-methylphenol

2-Amino-4-methylphenol (5.00g) and pyridine (6.6ml) were stirred in dichloromethane (100ml) in ice, as chloroacetyl chloride (3.4ml) was added dropwise. The mixture was stirred for 16h, when water (100 ml) was added. After stirring for a further 0.5h, the layers were separated. The aqueous portion was acidified (5M hydrochloric acid) and extracted with chloroform, and then filtered. The grey solid so isolated was washed with water and dried in vacuo . Recrystallisation from methanol/chloroform then gave the title compound (2.08g) as an off-white powder.
NMR (δ) ($DMSOd_6/CDCl_3$): 2.2 (3H,s), 4.25 (2H,s), 6.75(2H,m), 7.25(1H, b), 8.8-9.5(2H,b).

(b) 2,3-Dihydro-6-methyl-3-oxo-4H-1,4-benzoxazine

To a suspension of 2-(chloroacetylamino)-4-methylphenol (3.24g) in ethanol (35ml) was added sodium hydroxide (0.85g) in water (5ml). The mixture was swirled until homogeneous, left to stand for 2h, and diluted with water (500ml). Filtration and drying in vacuo then gave the title compund (2.41g) as a slightly pink solid.
NMR (δ) ($DMSOd_6$) 2.25(3H,s), 4.55(2H,s), 6.6-6.95(3H,m), 10.65(1H, b).

(c) Methyl 2,3-dihydro-6-methyl-3-oxo-4H-1,4-benzoxazine-4-acetate

2,3-Dihydro-6-methyl-3-oxo-4H-1,4-benzoxazine (2.70g), anhydrous potassium carbonate (3.43g) and methyl bromoacetate (2.0ml) were stirred in dry dimethylformamide (DMF) (20ml) for 16h. The mixture was diluted with ethyl acetate (200ml), washed with water and brine, dried ($Na_2SO_4$) and evaporated in vacuo . Recrystallisation from chloroform/petroleum ether (b.p.60-80°C) then gave the title compound (2.41g) as white needles.
NMR (δ) ($CDCl_3$): 2.3(3H,s), 3.75(3H,s), 4.4-4.8(4H,m), 6.4-6.9(3H,m).

(d) 2,3-Dihydro-6-methyl-3-oxo-4H-1,4-benzoxazine-4-acetic acid

This material was formed from methyl 2,3-dihydro-6-methyl-3-oxo-4H-1,4-benzoxazine-4-acetate (1.70g), following the procedure of Description 1(c), but with methanol as solvent. This gave the title compound (1.23g).
NMR (δ)($DMSOd_6$): 2.3(3H,s), 4.65(4H, 2xs), 6.7-7.0(3H,m).

Description 3

(a) BOC-Phe-Leu-ACHPA isobutylamide

BOC-Phe-Leu-OH (1.48g), 1-hydroxybenzotriazole (HOBT) (0.53g) and ACHPA isobutylamide (1.06g) [1] were stirred at 0°C in dry dimethylformamide (DMF) (10ml). 1-(3-Dimethylaminopropyl)-3-ethylcarbodiimide hydrochloride (DEC) (0.75g) was added, and the mixture was stirred for 18h, warming to ambient temperature. The mixture was diluted with ethyl acetate, washed with aqueous citric acid, water, aqueous sodium hydrogen carbonate and brine, dried ($Na_2SO_4$) and evaporated in vacuo . Chromatography on silica gel using methanol/chloroform (0-2% methanol, gradient) gave the title compound (1.91g).
NMR (δ) ($CDCl_3$): 0.7-2.3 (31 H,m) 2.8-3.2(4H,m), 3.8-4.3(4H,m), 6.4-7.4(8H,m).

(b) Phe-Leu-ACHPA isobutylamide .$CF_3CO_2H$

BOC-Phe-Leu-ACHPA isobutylamide (2.32g) was dissolved with stirring in trifluoroacetic acid (10ml) at 0°C. After 1h, solvent was removed in vacuo , and the residue was triturated with ether to give the title compound.
NMR (δ) ($CDCl_3$): 0.6-2.5 (31H,m), 2.8-3.3(4H,m), 3.7-4.4(4H,m), 7.0-8.0(8H,m).

Description 4

(a) CBZ-Phe-Leu-ACHPA isobutylamide

This material was formed from CBZ-Phe-Leu-OH (4.13g), following the procedure of Description 3(a). The crude product was purified by chromatography on silica gel using methanol/chloroform (0-3% methanol, gradient). This gave the title compound (6.40g) as a light brown solid.
NMR (δ) ($CDCl_3$): 0.75-1.85 (29H,m), 2.3(2H,m), 2.9-3.15 (4H,m), 4.0(2H,m), 4.3-4.5(2H,m), 5.05(2H,s), 5.5-(1H,d), 6.5-6.85(3H,m), 7.1-7.4(11H,m).

(b) Phe-Leu-ACHPA isobutylamide .$CH_3CO_2H$

This material was formed from CBZ-Phe-Leu-ACHPA isobutylamide (6.4g) following the procedure of Description 1(b), but using ethanol/acetic acid as solvent. This gave the title compound (7.4g), still

[1] J. Med. Chem. 1985, 28 , 1779-1790.

containing residual acetic acid.
NMR (δ) ($CDCl_3$): 0.8-1.85(29H,m), 2.05(m), 2.35(2H,m), 2.9-3.2(4H,m), 3.95(2H,m), 4.15(1H,m), 4.3(1H,m), 7.25(5H,m), 8.0(b).

(c) Phe-Leu-ACHPA isobutylamide

Phe-Leu-ACHPA isobutylamide .$CH_3CO_2H$ (7.4g) was dissolved in ethyl acetate and extracted into aqueous citric acid. The extract was basified with sodium carbonate, and the product was isolated by extraction into chloroform, drying ($Na_2SO_4$) and evaporation in vacuo . This gave the title compound (3.2g).
NMR (δ) ($CDCl_3$): 0.7-1.0 (13H,m), 1.0-1.9(16H,m), 2.3(2H,ABX), 2.75(1H,dd), 3.05(2H,m), 3.2(1H,dd), 3.65-(1H,dd), 3.95(2H,m), 4.35(1H,m), 6.5(1H,d), 6.6(1H,t), 7.15-7.35(6H,m), 7.7(1H,d).

Description 5

(a) Thiachroman-4-one 1,1-dioxide

Thiachroman-4-one (10.23g) was stirred in glacial acetic acid (50ml). Aqueous hydrogen peroxide (30%, 19.4ml) was added, and the mixture was brought to gentle reflux over 0.5h. After a further 0.5h at reflux, the mixture was cooled and poured into water (IL). The precipitate was filtered off, washed with water, and dried in vacuo , giving the title compound (10.00g) as a white solid.
NMR (δ) ($CDCl_3$): 3.4(2H,t), 3.7(2H,t), 7.8(2H,m), 8.0(1H,dd), 8.15(1H,dd).

(b) Ethyl thiachromanylidene-4-acetate 1,1-dioxide

Thiachroman-4-one 1,1-dioxide (2.44g) and (carbethoxymethylene)triphenylphosphorane (4.76g) were stirred at reflux in dry toluene (100ml) for 20h. Solvent was removed in vacuo , giving a brown solid, which was chromatographed on silica gel using ethyl acetate/petroleum ether (bp 60-80°C) (20-40% ethyl acetate, gradient). This gave the title compound (0.21g) as a yellow oil contaminated with ca 20% of starting material.
NMR (δ) ($CDCl_3$): 1.3(3H,t), 3.4(2H,t), 3.9(2H,td), 4.25(2H,q), 6.4(1H,s), 7.5-7.8(3H,m), 8.0(1H,m).

(c) Thiachromanylidene-4-acetic acid 1,1-dioxide

This material was formed from ethyl thiachromanylidene-4-acetate 1,1-dioxide (0.21g), following the procedure of Description 1(c). This gave the title compound (0.16g) as a yellow foam.
NMR (δ) ($DMSOd_6$) 3.4(m), 4.2(2H,m), 6.3(1H,t), 7.6-7.9(4H,m), 12.4(1H,b)

(d) Thiachromanyl-4-acetic acid 1,1-dioxide

This material was formed from thiachromanylidene-4-acetic acid 1,1-dioxide (0.11g), following the procedure of Description 1(b). This gave the title compound (0.09g) as a yellow foam.
NMR (δ) ($CD_3OD/CDCl_3$) 1.25(2H,m), 2.45(m), 2.7(2H,m), 3.4-3.7(m), 7.4-7.9(4H,m).

Description 6

(a) tert-Butyl (4-cyano-2-nitrophenoxy)acetate

4-Cyano-2-nitrophenol [2] (3.12g), sodium hydroxide (1.22g), benzyltributylammonium bromide (0.34g)

[2] Proc. Roy. Soc. (London) SerB, Vol 133, 30 (1946)

and tert-butyl bromoacetate (5.6ml) were stirred vigorously at reflux in a mixture of water (100ml) and 1,2-dichloroethane (100ml) for 16h. The mixture was cooled, acidified (5M hydrochloric acid) and separated. The aqueous portion was extracted with dichloromethane, and the combined organics were dried ($Na_2SO_4$) and evaporated in vacuo . Chromatography on silica gel using ethyl acetate/petroleum ether (bp 60-80°C) (10-40% ethyl acetate, gradient) gave the title compound (4.26g) as a cream solid, mp 121-3°C.
NMR (δ) ($CDCl_3$): 1.5(9H,s), 4.75(2H,s), 7.05(1H,d), 7.8(1H,dd), 8.2(1H,d).

(b) 6-Cyano-2,3-dihydro-3-oxo-4H-1,4-benzoxazine

tert-Butyl (4-cyano-2-nitrophenoxy)acetate (5.33g) was stirred in glacial acetic acid (100ml) at 40°C as iron (electrolytic, 4x3.2g portions) was added over 2h. After a further 1h, the mixture was evaporated in vacuo , and the residue was suspended in water (500ml). Filtration and drying in vacuo then gave the title compound (2.74g) as a grey solid.
NMR (δ) ($DMSOd_6$): 4.75(2H,s), 7.0-7.5(3H,m), 12.0(1H,b).

(c) Methyl 6-cyano-2,3-dihydro-3-oxo-4H-1,4-benzoxazine-4-propanoate

This material was formed from 6-cyano-2,3-dihydro-3-oxo-4H-1,4-benzoxazine (0.26g) and methyl acrylate (0.14ml), following the procedure of Description 2(c). This gave the title compound (0.35g) as a colourless oil.
NMR (δ) ($CDCl_3$): 2;7(2H,t) 3.7(3H,s), 4.25(2H,t), 4.7(2H,s), 7.05(1H,d), 7.3-7.4(2H,m).

(d) 6-Cyano-2,3-dihydro-3-oxo-4H-1,4-benzoxazine-4-propanoic acid

This material was formed from methyl 6-cyano-2,3-dihydro-3-oxo-4H-1,4-benzoxazine-4-propanoate (0.35g), following the procedure of Description 1(c). This gave the title compound (0.25g) as a white powder.
NMR (δ) ($CDCl_3/DMSOd_6$): 2.65(2H,t), 4.2(2H,t), 4.7(2H,s), 7.1(1H,d), 7.35(1H,dd), 7.55(1H,d).

Description 7

(a) Methyl 2,3-dihydro-3-oxo-4H-1,4-benzothiazine-4-propanoate

This material was formed from 2,3-dihydro-3-oxo-4H-1,4-benzothiazine (2.00g), following the procedure of Description 6(c). This gave the title compound (2.97g) as a green-brown oil.
NMR (δ) ($CDCl_3$): 2.7(2H,t), 3.4(2H,s), 3.7(3H,s), 4.3(2H,t), 7.05(1H,dt), 7.15(1H,dd), 7.25(1H,dt), 7.35(1H,dd).

(b) 2,3-Dihydro-3-oxo-4H-1,4-benzothiazine-4-propanoic acid

This material was formed from methyl 2,3-dihydro-3-oxo- 4H-1,4-benzothiazine-4-propanoate (0.19g), following the procedure of Description 1(c). This gave the title compound (0.14g) as a light yellow solid.
NMR (δ) ($CDCl_3/DMSOd_6$): 2.6(2H,t), 3.4(2H,s), 4.2(2H,t), 7.0(1H,m), 7.2-7.4(3H,m).

Description 8

(a) Methyl 2,3-dihydro-1,1,3-trioxo-4H-1,4-benzothiazine-4-propanoate

Methyl 2,3-dihydro-3-oxo-4H-1,4-benzothiazine-4-propanoate (0.10g) (Description 7(a)) and 3-chloroperoxybenzoic acid (50-55% grade, 0.29g) were stirred in dichloromethane (5ml) for 40 min. The mixture was then diluted with ethyl acetate (50ml), washed with 5% aqueous sodium hydrogen carbonate, dried ($Na_2SO_4$) and evaporated in vacuo . Chromatography on silica gel using ethyl acetate/petroleum ether

(b.p. 60-80°C) (20-100% ethyl acetate, gradient) then gave the title compound (0.10g) as a white solid. NMR (δ) ($CDCl_3$); 2.75(2H,t), 3.7(3H,s), 4.25(2H,s), 4.35(2H,t), 7.4(2H,m), 7.7(1H,dt), 8.0(1H,dd).

(b) 2,3-Dihydro-1,1,3-trioxo-4H-1,4-benzothiazine-4-propanoic acid

This material was formed from methyl 2,3-dihydro-1,1,3-trioxo-4H-1,4-benzothiazine-4-propanoate (0.10g), following the procedure of Description 1(c). This gave the title compound (0.05g) as a white solid. NMR (δ) ($CDCl_3$/$DMSOd_6$): 2.65(2H,t), 4.3(2H,t), 4.55(2H,s), 7.4(1H,t), 7.55(1H,d), 7.75(1H,t), 7.9(1H,d).

Description 9

a) BOC-ACHPA 3-(1-imidazolyl)propylamide

This material was formed from BOC-ACHPA-OH [3] (0.21g) and 1-(3-aminopropyl)imidazole (0.086 ml), following the procedure of Description 3(a), but omitting the citric acid wash. This gave the title compound (0.18g) as an oil.

b) CBZ-Phe-Leu-ACHPA 3-(1-imidazolyl)propylamide .0.5$H_2O$

BOC-ACHPA 3-(1-imidazolyl)propylamide (0.18g) was dissolved in dichloromethane (4 ml), and cooled in ice. TFA (4 ml) was added. The mixture was swirled and left to stand at ambient temperature for 0.5h, and then evaporated, dissolved in dry DMF (3 ml) and cooled in ice. CBZ-Phe-Leu-OH (0.19g) and HOBT (0.062g) were stirred at 0°C in dry DMF (3 ml), and DEC (0.088g) was added. This mixture was stirred at 0°C for 15 min, and then DIPEA (0.22 ml) and the amine trifluoroacetate solution, prepared above, were added. After stirring for 16h, with warming to ambient temperature, the work up procedure of Description 9-(a) was followed. The crude product was chromatographed on silica gel using methanol/chloroform (0-10% methanol, gradient). This gave the title compound (0.195g) as a white solid.
NMR (δ) ($CDCl_3$): 0.75-0.1 (7H, m), 1.05-2.5 (20H, m), 2.9-3.4 (4H, m), 3.9-4.1 (4H, m), 4.15-4.5 (2H, m), 5.0-5.15 (2H, m), 5.45 (b), 6.35-6.65 (2H, m), 6.9-7.1 (3H, m), 7.1-7.45 (10H, m), 7.5-7.8 (1H, m).
Analysis: $C_{40}H_{56}N_6O_6$.0.5$H_2O$ requires C,66.2; H,7.9; N,11.6%. Found: C,65.9; H,7.7; N,11.3%.
M.S. (m/z) (FAB) (M+1)=717 (consistent with m.w.=716).

c) Phe-Leu-ACHPA 3-(1-imidazolyl)propylamide

This material was formed from CBZ-Phe-Leu-ACHPA 3-(1-imidazolyl)propylamide (3.0g), following the procedure of Description 1(b). The crude product was chromatographed on silica gel using methanol/chloroform (0-16% methanol, gradient). This gave the title compound (1.4g) as a light yellow powder.
NMR (δ) ($DMSOd_6$): 0.7-1.9 (24H,m), 2.1 (2H,m), 2.55-2.7 (1H,m), 3.0 (3H,m), 3.8 (2H,m), 3.9 (2H,t), 4.3 (1H,q), 4.9 (1H,m), 6.85 (1H,b), 7.1-7.3 (6H,m), 7.4-7.8 (4H,m), 8.2 (1H,d).

Description 10

a) 6-(BOC-aminomethyl)-2,3-dihydro-3-oxo-4H-1,4-benzoxazine

6-Cyano-2,3-dihydro-3-oxo-4H-1,4-benzoxazine (0.18g) (Description 6(b)) was hydrogenated over platinum dioxide (0.05g) in ethyl acetate (25 ml) containing di-tert-butyldicarbonate (0.23g) for 70h. Catalyst was filtered off, and the filtrate was evaporated. The crude product was chromatographed on silica gel using ethyl acetate/petroleum ether (b.p. 60-80°C) (50-100% ethyl acetate, gradient). This gave the title compound

[3] J. Med. Chem. 1985, 28 , 1779-1790.

(0.18g) as a white solid.
NMR (δ) ($DMSOd_6$): 1.35(9H,s), 4.0(2H,d), 4.5(2H,s), 6.75(2H,m), 6.85(1H,d), 7.35(1H,t), 10.7(1H,s).

b) Methyl 6-(BOC-aminomethyl)-2,3-dihydro-3-oxo-4H-1,4-benzoxazine-4-propanoate

This material was formed from 6-(BOC-aminomethyl)-2,3-dihydro-3-oxo-4H-1,4-benzoxazine (0.17g), following the procedure of Description 6(c). This gave the title compound (0.24g) as a light yellow solid.
NMR (δ) ($CDCl_3$): 1.45(9H,s), 2.7(2H,t), 3.7(3H,s), 4.25(4H,m), 4.6(2H,s), 4.9(1H,b), 6.95(3H,m).

c) 6-(BOC-aminomethyl)-2,3-dihydro-3-oxo-4H-1,4-benzoxazine-4-propanoic acid

This material was formed from methyl 6-(BOC-aminomethyl)-2,3-dihydro-3-oxo-4H-1,4-benzoxazine-4-propanoate (0.23g), following the procedure of Description 1(c). This gave the title compound (0.19g) as a light yellow solid.
NMR (δ) ($CDCl_3$): 1.45(9H,s), 2.75(2H,t), 4.25(2H,m), 4.6(2H,s), 5.05(1H,b), 6.9(2H,m), 7.05(1H,b).

Description 11

3-(1,2,3,4-Tetrahydroisoquinolin-2-yl)propanoic acid

This material ws formed from ethyl 3-(1,2,3,4-tetrahydroisoquinolin-2-yl)propanoate [4](3.00g) following the procedure of Description 1(c). Removal of the solvent from the acidified reaction mixture gave an orange oil which was triturated with methanol/chloroform to give the title compound (2.69g) as a purple semi-solid.
NMR (δ) ($DMSOd_6$): 2.4-2.5(2H,m), 2.6-2.8(6H,m), 3.6(2H,bs), 7.0-7.2(4H,m).

Description 12

a) BOC-Phe-Leu-ACHPA 3-(1-imidazolyl)propylamide

This material was formed from BOC-ACHPA 3-(1-imidazolyl)propylamide (1.53g) (Description 9(a)) and BOC-Phe-Leu-OH (1.60g), following the procedure of Description 9(b). The crude product was chromatographed on silica gel using methanol/chloroform (0-10% methanol, gradient). This gave the title compound (1.95g).
NMR (δ) ($CDCl_3$): 0.7-1.05(8H,m), 1.05-1.9(2H,m), 2.0(2H,m), 2.2-2.5(2H,m), 2.9-3.6(5H,m), 3.9-4.5(6H,m), 5.1(b), 6.4(b), 6.6(bd), 7.0(1H,s), 7.05(1H,s), 7.15-7.4(m), 7.65(1H,s).

b) Phe-Leu-ACHPA 3-(1-imidazolyl)propylamide .2($CF_3CO_2H$)

To a solution of BOC-Phe-Leu-ACHPA 3-(1-imidazolyl)-propylamide (0.60g) in dichloromethane (6 ml) was added trifluoroacetic acid (6 ml). The mixture was stirred for 1 h, and evaporated. Trituration with ether/pentane and filtration then gave the title compound (0.62g) as a white solid.

Description 13

a) tert-Butyl (4-(tert-butoxycarbonylmethoxycarbonyl)-2-nitrophenoxy)acetate

[4]Coll. Czech. Chem. Commun. 43 , (1983), 1759.

4-Hydroxy-3-nitrobenzoic acid (5.00g), tert-butyl bromoacetate (11.8 ml), sodium hydroxide (2.57g) and benzyltributylammonium bromide (0.52g) were stirred at reflux in water (50 ml) and 1,2-dichloroethane (50 ml) for 16h, then cooled to room temperature. After separation, the aqueous phase was acidified to pH 0 (5M hydrochloric acid) and extracted with chloroform (100 ml). The combined organics were dried ($Na_2SO_4$) and solvents removed in vacuo . The crude product was dissolved in ether, washed with 10% sodium hydroxide solution, dried ($Na_2SO_4$), and solvent was removed in vacuo . The crude product (9.46g) was purified by chromatography on silica gel using ethyl acetate/petroleum ether (b.p. 60-80°C) to give the title compound (8.03g) as a slightly coloured oil.

NMR (δ) ($CDCl_3$): 1.5 (18H,2xs), 4.7 (4H,s), 7.0 (1H,dd), 8.2 (1H,dd), 8.6 (1H,d).

b) tert-Butyl (2-amino-4-(tert-butoxycarbonylmethoxycarbonyl)phenoxy)acetate

This material was formed from tert-butyl (4-(tertbutoxycarbonylmethoxycarbonyl)-2-nitrophenoxy)acetate (4.20g) following the procedure of Description 1(b). This gave the title compound (3.96g) as a semi-solid.

NMR (δ) ($CDCl_3$): 1.5 (18H,s), 4.6 (2H,s), 4.7 (2H,s), 6.7 (1H,m), 7.5 (2H,m).

c) 6-(tert-Butoxycarbonylmethoxycarbonyl)-2,3-dihydro-3-oxo-4H-1,4-benzoxazine

tert-Butyl (2-amino-4-(tert-butoxycarbonylmethoxycarbonyl)phenoxy)acetate (3.96g) was heated at reflux in xylene for 48 h. After cooling to room temperature; solvent was removed in vacuo . The residues were triturated with petroleum ether (b.p. 60-80°C) to give the title compound (2.22g) as a light grey solid.

NMR (δ) ($CDCl_3$): 1.5 (9H,s), 4.7 (4H,2xs), 7.0 (1H,d), 7.6 (1H,d), 7.8 (1H,dd), 8.5 (1H,bs).

d) 6-Carboxy-2,3-dihydro-3-oxo-4H-1,4-benzoxazine

6-(tert-Butoxycarbonylmethoxycarbonyl-2,3-dihydro-3-oxo-4H-1,4-benzoxazine (2.11g) was stirred in ethanol (15 ml) with 10% sodium hydroxide solution (6.8 ml). The solution was diluted with water (50 ml) and washed with chloroform (2x20 ml), acidified to pH 0 (5M hydrochloric acid), then extracted with chloroform (3x20 ml) and ethyl acetate (2x20 ml). The combined organics were dried ($Na_2SO_4$) and solvents removed in vacuo to give the title compound (0.80g) as a white solid.

NMR (δ) ($DMSOd_6$): 4.6 (2H,s), 7.0 (1H,m), 7.5 (2H,m), 10.9 (1H,bs).

e) 6-Benzyloxycarbonyl-2,3-dihydro-3-oxo-4H-1,4-benzoxazine

6-Carboxy-2,3-dihydro-3-oxo-4H-1,4-benzoxazine (0.17g) was stirred with DEC (0.17g), benzyl alcohol (0.1 ml) and 4-dimethylaminopyridine (0.012g) in DMF (5 ml) for 16h. The solution was diluted with ethyl acetate (50 ml) and washed with water (3x15 ml). The combined organics were dried ($Na_2SO_4$) and solvents removed in vacuo to give the title compound (0.21g).

NMR (δ) ($CDCl_3$): 4.7 (2H,s), 5.35 (2H,s), 7.0 (1H,d), 7.3-7.5 (5H,m), 7.6 (1H,d), 7.7 (1H,dd), 8.4 (1H,bs).

f) 6-Benzyloxycarbonyl-2,3-dihydro-3-oxo-4H-1,4-benzoxazine-4-propanoic acid

This material was formed from 6-benzyloxycarbonyl-2,3-dihydro-3-oxo-4H-1,4-benzoxazine (0.85g) and tert-butyl acrylate (0.81g), following the procedure of Description 6(c). The crude product was stirred for 3h in dichloromethane (10 ml) and trifluoroacetic acid (10 ml). Removal of solvents gave the title compound (0.59g) as a white solid.

NMR (δ) ($DMSOd_6$): 2.6 (2H,t), 4.2 (2H,t), 4.7 (2H,s), 5.4 (2H,s), 7.1 (1H,m), 7.3-7.5 (5H,m), 7.7 (1H,m), 7.8 (1H,m).

Description 14

a) tert-Butyl (4-(tert-butoxycarbonylmethoxycarbonylmethyl)-2-nitrophenoxy)acetate

This material was formed from 4-hydroxy-3-nitrophenylacetic acid (1.00g) following the procedure of Description 13(a). The crude product was purified by column chromatography on silica gel using ethyl acetate/petroleum ether (b.p. 60-80°C) (0-30% ethyl acetate, gradient). The title compound (1.85g) was isolated as an oil.
NMR (δ) ($CDCl_3$): 1.5 (18H,2xs), 3.7 (2H,s), 4.5 (2H,s), 4.6 (2H,s), 6.9 (1H,d), 7.5 (1H,dd), 7.8 (1H,d).

b) tert-Butyl (2-amino-4-(tert-butoxycarbonylmethoxycarbonylmethyl)phenoxy)acetate

This material was formed from tert-butyl(4-(tert-butoxycarbonylmethoxycarbonylmethyl)-2-nitrophenoxy)-acetate (1.85g) following the procedure of Description 1(b). This gave the title compound (1.50g) as a semi-solid.
NMR (δ) ($CDCl_3$): 1.5 (18H,2xs), 3.6 (2H,s), 4.5 (4H,2xs), 6.7 (2H,m), 6.9 (1H,s).

c) 6-(tert-Butoxycarbonylmethoxycarbonylmethyl)-2,3-dihydro-3-oxo-4H-1,4-benzoxazine

This material was formed from tert-butyl (2-amino-4-(tert-butoxycarbonylmethoxycarbonylmethyl)-phenoxy)acetate (1.50g) following the procedure of Description 13(c) but with a reaction time of 16h. The title compound (1.04g) was isolated as a white solid.
NMR (δ) ($CDCl_3$): 15 (9H,s), 3.7 (1H,s), 4.5 (2H,s), 4.6 (2H,s), 6.8 (1H,s), 6.9 (1H,m).

d) 6-(Carboxymethyl)-2,3-dihydro-3-oxo-4H-1,4-benzoxazine

This material was formed from 6-(tert-butoxycarbonylmethoxycarbonylmethyl)-2,3-dihydro-3-oxo-4H-1,4-benzoxazine (1.04g) following the procedure of Description 13(d). The title compound (0.39g) was isolated as a semi-solid.
NMR (δ) ($DMSOd_6$): 3.5 (2H,s), 4.6 (2H,s), 6.8 (2H,m), 6.9 (1H,m), 10.7 (1H,bs), 12.4 (1H,bs).

e) 6-(Benzyloxycarbonylmethyl)-2,3-dihydro-3-oxo-4H-1,4-benzoxazine

This material was formed from 6-(carboxymethyl)-2,3-dihydro-3-oxo-4H-1,4-benzoxazine (0.39g) following the procedure of Description 13(e). The title compound (0.36g) was isolated as an oil.
NMR (δ) ($CDCl_3$): 3.6 (2H,s), 4.6 (2H,s), 5.2 (2H,s), 6.8 (1H,m), 6.9 (2H,m), 7.3-7.4 (5H,m), 8.5 (1H,bs).

f) 6-(Benzyloxycarbonylmethyl)-2,3-dihydro-3-oxo-4H-1,4-benzoxazine-4-propanoic acid

This material was formed from 6-(benzyloxycarbonylmethyl)-2,3-dihydro-3-oxo-4H-1,4-benzoxazine (0.36g) following the procedure of Description 13(f). The title compound (0.11g) was isolated as a white solid.
NMR (δ) ($CDCl_3$): 2.7 (2H,t), 3.6 (2H,s), 4.2 (2H,t), 4.6 (2H,s), 5.1 (2H,s), 6.9 (3H,m), 7.2-7.4 (5H,m).

Description 15

a) tert-Butyl 3,4-dihydro-4-oxo-1,5-benzothiazepine-5(2H)-propanoate

This material was formed from 2,3-dihydro-1,5-benzothiazepin-4(5H)one [5] (0.62g) and tert-butyl acrylate (2 ml) following the procedure of Description 6(c), giving the title compound (0.68g) as a white solid.
NMR (δ) ($CDCl_3$): 1.3 (9H,s), 2.4-2.6 (3H,m), 2.6-2.8 (1H,m), 3.3-3.4 (2H,m), 3.6-3.7 (1H,m), 4.4-4.6 (1H,m),

[5] Synth . Commun . 15(7), 623.

7.2 (1H,m), 7.3 (1H,dd), 7.5 (1H,m), 7.6 (1H,dd).

b) 3,4-Dihydro-4-oxo-1,5-benzothiazepine-5(2H)-propanoic acid

tert-Butyl 3,4-dihydro-4-oxo-1,5-benzothiazepine-5(2H)-propanoate (0.13g) was stirred in trifluoroacetic acid (20 ml) and dichloromethane (20 ml) for 3h. Removal of the solvent followed by trituration with ether gave the title compound (0.092g) as a white solid.
NMR (δ) ($CDCl_3$): 2.4-2.7 (3H,m), 2.7-3.0 (1H,m), 3.2-3.4 (2H,m), 3.6-3.8 (1H,m), 4.4-4.6 (1H,m), 7.2-7.3 (2H,m), 7.4-7.5 (1H,m), 7.6 (1H,m).

Description 16

a) tert-Butyl 3,4-dihydro-1,1,4-trioxo-1,5-benzothiazepine-5(2H)-propanoate

tert-Butyl 3,4-dihydro-4-oxo-1,5-benzothiazepine-5(2H)-propanoate (Description 15(a)) (0.52g) was stirred with 3-chloroperoxybenzoic acid (MCPBA) (70%, 0.83g) in dry dichloromethane for 16h. Solvent was removed in vacuo to give the crude product. This was purified by column chromatography on silica gel using ethyl acetate/petroleum ether (b.p. 60-80°C) (0-65% ethyl acetate, gradient) to give the title compound (0.45g) as a white solid.
NMR (δ) ($CDCl_3$): 1.4(9H,s), 2.5-2.9(4H,m), 3.4-3.6(1H,m), 3.8-3.9(2H,m), 4.4-4.5(1H,m), 7.4-7.5(2H,m), 7.7-7.8(1H,m), 8.0-8.1(1H,dd).

b) 3,4-Dihydro-1,1,4-trioxo-1,5-benzothiazepine-5(2H)-propanoic acid

This material was formed from tert-butyl 3,4-dihydro-1,1,4-trioxo-1,5-benzothiazepine-5(2H)-propanoate (0.45g), following the procedure of Description 15(b). The title compound (0.3g) was isolated as a white solid.
NMR (δ) ($DMSOd_6$): 2.5-2.7(m), 3.6-3.9(3H,m), 4.1-4.3(1H,m), 7.5-7.6(1H,m), 7.7-7.9(3H,m).

Description 17

a) Ethyl (4-cyano-2-nitrophenyl)thioacetate

This material was formed from 4-chloro-3-nitrobenzonitrile (5.00g) and ethyl mercaptoacetate (4.50 ml), following the procedure of Description 2(c). This gave the title compound (6.98g) as a green solid.
NMR (δ) ($CDCl_3$): 1.3(3H,t), 3.8(2H,s), 4.2(2H,q), 7.7(1H,d), 7.8(1H,dd), 8.6(1H,d).

b) 6-Cyano-2,3-dihydro-3-oxo-4H-1,4-benzothiazine

Ethyl (4-cyano-2-nitrophenyl)thioacetate (2.6g) was refluxed with iron powder (2.01g) in acetic acid (100 ml) for 1h. After cooling to room temperature the solution was poured onto ice (200 ml), then extracted with chloroform (5x50 ml). The combined organic phases were washed with sodium hydrogen carbonate solution (3x50 ml), dried ($Na_2SO_4$), and solvents were removed in vacuo . The title compound (1.52g) was isolated as a pale yellow solid.
NMR (δ) ($DMSOd_6/CDCl_3$): 3.35(2H,s), 7.1(1H,dd), 7.2(1H,d), 7.3(1H,d), 10.7(1H,bs).

c) Methyl 6-cyano-2,3-dihydro-3-oxo-4H-1,4-benzothiazine-4-propanoate

This material was formed from 6-cyano-2,3-dihydro-3-oxo-4H-1,4-benzothiazine (0.57g), following the procedure of Description 6(c). This gave the title compound (0.75g) as a yellow solid.

NMR (δ) ($CDCl_3$): 2.7(2H,t), 3.4(2H,s), 3.7(3H,s), 4.3(2H,t), 7.3(1H,dd), 7.4(1H,d), 7.5(1H,d).

d) Methyl 6-(BOC-aminomethyl)-2,3-dihydro-3-oxo-4H-1,4-benzothiazine-4-propanoate

Methyl 6-cyano-2,3-dihydro-3-oxo-4H-1,4-benzothiazine-4-propanoate (0.75g) was dissolved with heating in methanol (10 ml), and stirred as cobalt (II) chloride.$6H_2O$ (1.29g) and sodium borohydride (0.46g) were added. The resultant black suspension was stirred for 30 min, partitioned between chloroform and water, acidified with 5M hydrochloric acid, basified with aqueous ammonia (sp.gr.0.88), and separated. The aqueous portion was further extracted with chloroform, and the combined organics were dried ($Na_2SO_4$) and evaporated in vacuo.

Di-tert-butyldicarbonate (0.59g) was added, and the mixture was dissolved in chloroform (10 ml), left to stand for 16h, and evaporated in vacuo . Chromatography on silica gel using ethyl acetate/petroleum ether (b.p. 60-80°C) (20-60% ethyl acetate, gradient) then gave the title compound (0.32g) as a yellow solid.
NMR (δ) ($CDCl_3$): 1.45(9H,s), 2.7(2H,t), 3.35(2H,s), 3.65(3H,s), 4.3(4H,m), 4.95(1H,b), 6.95(1H,dd), 7.1(1H,d), 7.3(1H,d).

e) 6-(BOC-aminomethyl)-2,3-dihydro-3-oxo-4H-1,4-benzothiazine-4-propanoic acid

This material was formed from methyl 6-(BOC-aminomethyl)-2,3-dihydro-3-oxo-4H-1,4-benzothiazine-4-propanoate (0.30g), following the procedure of Description 1(c). The crude product was dissolved in ether, filtered, evaporated, and triturated in 10% ethyl acetate/petroleum ether (b.p. 60-80°C), giving the title compound (0.27g) as a yellow powder.
NMR (δ) ($CDCl_3$): 1.45(9H,s), 2.75(2H,t), 3.4(2H,s), 4.3(4H,m), 6.95(1H,bm), 7.15(1H,bs), 7.3(1H,d).

Example 1

Chromanyl-4-acetyl-Phe-Leu-ACHPA isobutylamide .$0.5H_2O$

Chromanyl-4-acetic acid (0.052g) and 1-hydroxybenzotriazole (HOBT) (0.040g) were stirred in dry dimethylformamide (DMF) (3ml) in ice. 1-(3-Dimethylaminopropyl)-3-ethylcarbodiimide hydrochloride (DEC) (0.052g) was added, and the mixture was stirred for 15 min. N,N-Diisopropylethylamine (DIPEA) (0.12ml) and Phe-Leu-ACHPA isobutylamide .$CH_3CO_2H$ (0.129g) (Description 4(b)) were added. The mixture was stirred for 16h, warming to ambient temperature, diluted with ethyl acetate, washed with 5% aqueous citric acid, water, 5% aqueous sodium hydrogen carbonate and brine, dried ($Na_2SO_4$) and evaporated in vacuo . Chromatography on silica gel using methanol/chloroform (0-5% methanol, gradient) gave the title compound (0.076g) as a white solid.
NMR (δ) (DMSOd$_6$): 0.7-1.0(13H,m), 1.0-1.4(7H,m), 1.4-1.8(10H,m), 2.0-2.25(3H,m), 2.5(m), 2.65-3.1(6H,m), 3.75-4.1(4H,m), 4.35(1H,m), 4.65(1H,m), 4.8-5.0(1H,m), 6.7(1H,dd), 6.75(1H,m), 7.05(2H,m), 7.1-7.4(6H,m), 7.7(1H,m), 8.1-8.3(2H,m).
Analysis: $C_{41}H_{60}N_4O_6.0.5H_2O$ required C, 69.0; H, 8.6; N, 7.8%. Found: C, 69.3; H, 8.5; N, 7.8%
M.S. (m/z) (FAB) (M + 1) = 705 (consistent with m.w. = 704).

Example 2

2,3-Dihydro-6-methyl-3-oxo-4H-1,4-benzoxazine-4-acetyl-Phe-Leu-ACHPA isobutylamide .$H_2O$

This material was formed from 2,3-dihydro-6-methyl-3-oxo-4H-1,4-benzoxazine-4-acetic acid (0.041g) and Phe-Leu-ACHPA isobutylamide .$CF_3CO_2H$ (0.100g) (Description 3(b)), following the procedure of Example 1. The crude product was chromatographed on silica gel using methanol/chloroform (0-5% methanol, gradient). This gave the title compound (0.107g) as a white solid.
NMR (δ) (DMSOd$_6$): 0.7-1.0(13H,m), 1.0-1.8(16H,m), 2.1(2H,m), 2.2(3H,s), 2.7-3.1(4H,m), 3.75-3.9(2H,m), 4.3-4.65(6H,m), 4.8-4.9(1H,m), 6.5-6.6(1H,m), 6.75(1H,d), 6.85(1H,d), 7.15-7.55(6H,m), 7.7(1H,m), 8.15-8.25-

(1H,m), 8.45-8.55(1H,m).
Analysis: $C_{41}H_{59}N_5O_7.H_2O$ requires C,65.5; H, 8.2; N, 9.3%. Found: C, 65.4; H, 7.9; N, 9.3%.
M.S. (m/z) (FAB) (M+1) 734 (consistent with m.w. = 733).

Example 3

1,1-Dioxothiachromanylidene-4-acetyl-Phe-Leu-ACHPA isobutylamide .0.5$H_2O$

This method was formed from thiachromanylidene-4-acetic acid 1,1-dioxide (0.050g) (Description 5(c)) and Phe-Leu-ACHPA isobutylamide (0.098g) (Description 4(c)), following the procedure of Description 3(a). The crude product was chromatographed on silica gel using methanol/chloroform (0-5% methanol, gradient). This gave the title compound (0.100g) as a yellow-brown solid.
NMR (δ) (DMSO$d_6$): 0.7-1.0(14H,m), 1.0-1.8(15H,m), 2.1(2H,m), 2.7-2.9(4H,m), 3.05(1H,dd), 3.4(2H,m), 3.8-(2H,m), 4.1(2H,m), 4.3(1H,m), 4.6(1H,m), 4.9(1H,m), 6.0(1H,t), 7.1-7.4(7H,m), 7.55(2H,m), 7.7(1H,bs), 7.85-(1H,m), 8.2(1H,bd), 8.35(1H,bd).
Analysis: $C_{41}H_{58}N_4O_7S.0.5H_2O$ requires C,64.8; H,7.8; N,7.4%. Found: C,64.8; H,7.8; N,7.2%.
M.S. (m/z) (FAB) (M+1) = 751 (consistent with m.w. = 750).

Example 4

1,1-Dioxothiachromanyl-4-acetyl-Phe-Leu-ACHPA isobutylamide .0.5$H_2O$

This material was formed from thiachromanyl-4-acetic acid 1,1-dioxide (0.068g) (Description 5(d)), following the procedure of Example 3. The crude product was chromatographed on silica gel using methanol/chloroform (0-5% methanol, gradient). This gave the title compound (0.075g) as a yellow solid.
NMR (δ) (DMSO$d_6$): 0.7-1.0(13H,m), 1.0-1.4(7H,m), 1.4-1.8(10H,m), 1.9-2.25(3H,m), 2.4-2.6(m), 2.7(1H,m), 2.9(2H,m), 3.0-3.2(1H,m), 3.2-3.6(m), 3.85(2H,m), 4.3(1H,m), 4.65(0.5H,m), 4.75(0.5H,m), 4.9(1H,m), 7.15-7.4(7H,m) 7.4-7.6(2H,m), 7.6-7.8(2H,m), 8.1-8.4(2H,m).
Analysis: $C_{41}H_{60}N_4O_7S.0.5H_2O$ requires C,64.6; H,8.1; N,7.4%. Found C,64.5; H,8.0; N,7.5%.
M.S. (m/z) (FAB) (M+1) 753 (consistent with m.w. = 752).

Example 5

6-Cyano-2,3-dihydro-3-oxo-4H-1,4-benzoxazine-4-propanoyl-Phe-Leu-ACHPA isobutylamide .0.5$H_2O$

This material was formed from 6-cyano-2,3-dihydro-3-oxo-4H-1,4-benzoxazine-4-propanoic acid (0.078g) (Description 6(d)), following the procedure of Description 3(a). The crude product was chromatographed on silica gel using methanol/chloroform (0-5% methanol, gradient). This gave the title compound (0.21g) as a white solid.
NMR (δ) (DMSO$d_6$): 0.7-1.0(13H,m), 1.0-1.45(7H,m), 1.45-1.8(9H,m), 2.1(2H,m), 2.4(2H,t), 2.7(1H,dd), 2.85-(2H,m), 3.0(1H,dd), 3.8(3H,m), 4.0(1H,m), 4.3(1H,m), 4.6(1H,m), 4.75(2H,s), 4.9(1H,d), 7.1-7.35(7H,m), 7.5-(1H,dd), 7.6(1H,d), 7.7(1H,t), 8.15(1H,d), 8.25(1H,d).
Analysis: $C_{42}H_{58}N_6O_7.0.5H_2O$ requires C,65.7; H,7.7; N,10.9%. Found: C,65.8; H,7.8; N,11.3%.
M.S. (m/z) (FAB) (M+1) = 759 (consistent with m.w. = 758).

Example 6

2,3-Dihydro-3-oxo-4H-1,4-benzothiazine-4-propanoyl-Phe-Leu-ACHPA isobutylamide .0.5$H_2O$

This material was formed from 2,3-dihydro-3-oxo-4H-1,4-benzothiazine-4-propanoic acid (0.051g)

(Description 7(b)), following the procedure of Description 3(a). The crude product was chromatographed on silica gel using methanol/chloroform (0-5% methanol, gradient). This gave the title compound (0.11g) as a white solid.

NMR (δ) (DMSOd$_6$): 0.7-10(13H,m), 1.0-1.4(7H,m), 1.4-1.8(9H,m), 2.1(2H,m), 2.4(2H,m), 2.7(1H,dd), 2.85-(2H,m), 3.0(1H,dd), 3.45(2H,s), 3.8-3.9(3H,m), 4.0(1H,m), 4.3(1H,m), 4.55(1H,m) 4.85(1H,d), 7.05(1H,m), 7.1-7.35(8H,m), 7.4(1H,d), 7.7(1H,t), 8.1(1H,d), 8.25(1H,d).

Analysis: $C_{41}H_{59}N_5O_6S.0.5H_2O$ requires C,64.9; H,8.0; N,9.2%. Found C,64,8; H,8.0; N,9.1%.

M.S. (m/z) (FAB) (M+1) = 750 (consistent with m.w. = 749).

## Example 7

2,3-Dihydro-1,1,3-trioxo-4H-1,4-benzothiazine-4-propanoyl-Phe-Leu-ACHPA isobutylamide .0.5H$_2$O

This material was formed from 2,3-dihydro-1,1,3-trioxo-4H-1,4-benzothiazine-4-propanoic acid (0.044g) (Description 8(b)), following the procedure of Description 3(a). The crude product was chromatographed on silica gel using methanol/chloroform (0-5% methanol, gradient). This gave the title compound (0.076g) as a white solid.

NMR (δ) (DMSOd$_6$): 0.7-1.0(13H,m), 1.0-1.8(16H,m), 2.1(2H,m), 2.4(2H,m), 2.7(1H,dd), 2.85(2H,m), 3.0-(1H,dd), 3.8(2H,m), 3.95(1H,m), 4.1(1H,m), 4.3(1H,m), 4.55(1H,m), 4.75(2H,s), 4.85(1H,d), 7.1-7.25(5H,m), 7.3(1H,d), 7.4(1H,t), 7.55(1H,d), 7.7(1H,t), 7.75(1H,t), 7.9(1H,d), 8.15(1H,d), 8.3(1H,d).

Analysis: $C_{41}H_{59}N_5O_8S.0.5H_2O$ requires C,62.3; H,7.7; N,8.9%. Found C,62.3; H,7.7; N,8.6%.

M.S. (m/z) (FAB) (M+1) = 782 (consistent with m.w. = 781).

## Example 8a

2,3-Dihydro-1,1,3-trioxo-4H-1,4-benzothiazine-4-propanoyl-Phe-Leu-ACHPA 3-(1-imidazolyl)propylamide .1.5H$_2$O

This material was formed from 2,3-dihydro-1,1,3-trioxo-4H-1,4-benzothiazine-4-propanoic acid (0.048g) (Description 8(b)) and Phe-Leu-ACHPA 3-(1-imidazolyl)propylamide .2CF$_3$CO$_2$H (0.084g) (Description 12(b)-), following the procedure of Example 1, but omitting the wash with citric acid. The crude product was chromatographed on silica gel using methanol/chloroform (0-10% methanol, gradient). This gave the title compound (0.078g) as a white solid.

NMR (δ) (DMSOd$_6$): 0.7-1.0 (8H,m), 1.0-1.85 (16H,m), 2.0-2.25 (2H,m), 2.4 (2H,m), 2.75 (1H,m), 2.9-3.1 (3H,m), 3.85 (2H,b), 3.9-4.2 (4H,m), 4.2-4.35 (1H,m), 4.55 (1H,m), 4.75-4.9 (3H,m), 6.85 (1H,2xs), 7.1-7.25 (6H,m), 7.25-7.6 (4H,m), 7.75 (2H,m), 7.9 (1H,d), 8.2 (1H,2xd), 8.3 (d) and 8.45 (d)(1H).

Analysis: $C_{43}H_{59}N_7O_8S.1.5H_2O$ requires C,60.0; H,7.3; N,11.4%. Found: C,60.1; H,7.1; N,11.1%.

M.S. (m/z) (FAB) (M+1) = 834 (consistent with m.w. = 833).

## Example 8b

2,3-Dihydro-1,1,3-trioxo-4H-1,4-benzothiazine-4-propanoyl-Phe-Leu-ACHPA 3-(1-imidazolyl)propylamide .HCl

The corresponding free base (Example 8a) (0.065g) was dissolved in methanol (2 ml), acidified with a slight excess of 5M hydrochloric acid, diluted with water (40 ml), and immediately freeze-dried. This gave the title compound (0.065g) as a light white powder.

## Example 9

2,3-Dioxo-1,2,3,4-tetrahydroquinoxaline-1-acetyl-Phe-Leu-ACHPA isobutylamide $.H_2O$

This material was formed from 3-oxo-1,2,3,4-tetrahydroquinoxaline-1-acetic acid (0.043g) [6], following the procedure of Example 1. The crude product was chromatographed on silica gel using methanol/chloroform (0-15% methanol, gradient). This gave the title compound (0.050g) as a light yellow solid.
NMR (δ) ($DMSOd_6$): 0.7-1.0 (13H,m), 1.0-1.4 (7H,m), 1.4-1.8 (9H,m), 2.1 (2H,m), 2.75 (1H,dd), 2.85 (2H,m), 3.05 (1H,dd), 3.8 (2H,m), 4.3 (1H,q), 4.6 (2H,m), 4.8 (1H,d), 4.9 (1H,d), 6.65 (1H,d), 7.0 (1H,m), 7.1-7.3 (7H,m), 7.35 (1H,d), 7.7 (1H,t), 8.2 (1H,d), 8.6 (1H,d), 12.05 (1H,b).
Analysis: $C_{40}H_{56}N_6O_7.H_2O$ requires C,64.0; H,7.8%. Found: C,64.0; H,7.7%.
M.S. (m/z) (FAB) (M+1) 733 (consistent with m.w. = 732).

Example 10

6-(BOC-aminomethyl)-2,3-dihydro-3-oxo-4H-1,4-benzoxazine-4-propanoyl-Phe-Leu-ACHPA 3-(1-imidazolyl)-propylamide .0.5 $CHCl_3$

This material was formed from 6-(BOC-aminomethyl)-2,3-dihydro-3-oxo-4H-1,4-benzoxazine-4-propanoic acid (0.15g) (Description 10(c)), following the procedure of Example 8a. The crude product was chromatographed on silica gel using methanol/chloroform (0-10% methanol, gradient). This gave the title compound (0.21g) as a light yellow solid.
NMR (δ) ($DMSOd_6$): 0.8 (2H,m), 0.9 (6H,2xdd), 1.0-1.45 (14H,m), 1.45-1.9 (11H,m), 2.1 (2H,m), 2.4 (2H,t), 2.75 (1H,dd), 2.9-3.1 (3H,m), 3.75-3.9 (3H,m), 3.9-4.05 (3H,m), 4.1 (2H,d), 4.3 (1H,q), 4.5-4.65 (3H,m), 4.9 (1H,d), 6.85 (2H,m), 6.95 (1H,m), 7.0 (1H,s), 7.15 (2H,m), 7.25 (4H,m), 7.35 (2H,m), 7.6 (1H,s), 7.75 (1H,t), 8.2 (1H,d), 8.25 (1H,d), 8.3 (1H,s).
Analysis: $C_{49}H_{70}N_8O_9.0.5\ CHCl_3$ requires C,61.0; H,7.3; N,11.5%. Found: C,60.9; H,7.3; N,11.5%.
M.S. (m/z) (FAB) (M+1) 915 (consistent with m.w. = 914).

Example 11

6-(Aminomethyl)-2,3-dihydro-3-oxo-4H-1,4-benzoxazine-4-propanoyl-Phe-Leu-ACHPA 3-(1imidazolyl)-propylamide $.3CF_3CO_2H$

6-(BOC-aminomethyl)-2,3-dihydro-3-oxo-4H-1,4-benzoxazine-4-propanoyl-Phe-Leu-ACHPA 3-(1-imidazolyl)propylamide (0.19g) (Example 10) was dissolved in dichloromethane (3 ml). TFA (3 ml) was added, and the mixture was left to stand for 0.5h, evaporated, dissolved in water (25 ml), and freeze-dried. This gave the title compound (0.22g) as a fluffy white solid.
NMR (δ) ($DMSOd_6$): 0.7-1.0 (8H,m), 1.0-1.45 (7H,m), 1.45-1.7 (6H,m), 1.75 (1H,d), 1.9 (2H,m), 2.1 (2H,m), 2.45 (2H,m), 2.7 (1H,dd), 3.0 (3H,m), 3.85 (3H,m), 4.0 (3H,m), 4.2 (2H,t), 4.3 (1H,q), 4.55 (1H,m), 4.65 (2H,s), 4.9 (1H,b), 7.05 (2H,m), 7.1-7.3 (6H,m), 7.4 (1H,d), 7.7 (1H,s), 7.75 (1H,s), 7.85 (1H,t), 8.15 (3H,b), 8.25 (2H,t), 9.05 (1H,s), 14.5 (b).
Analysis: $C_{44}H_{62}N_8O_7.3C_2HO_2F_3$ requires C,51.9; H,5.7; N,9.7%. Found: C,52.3; H,5.8; N,9.8%.
M.S. (m/z) (FAB) (M+1) = 815 (consistent with m.w. of free base = 814).

Example 12

3-(1,2,3,4-Tetrahydroisoquinolin-2-yl)propanoyl-Phe-Leu-ACHPA 3-(1-imidazolyl)propylamide

This material was formed from 3-(1,2,3,4-tetrahydroisoquinolin-2-yl)propanoic acid (Description 11) (0.077g) following the procedure of Example 8a. The crude product was purified by chromatograpy on silica gel using methanol/chloroform (0-10% methanol, gradient) to give the title compound (0.11g) as a white

[6] N. Borthakur et al., Ind. J. Chem. 20B, 822 (1981).

solid.
NMR (δ) ($DMSOd_6$): 0.7-0.9 (8H,m), 1.0-1.7 (16H,m), 1.7-1.9 (4H,m), 2.0-2.2 (2H,m), 2.3 (2H,t), 2.5-2.8 (6H,m), 2.9-3.1 (3H,m), 3.5 (2H,m), 3.9-4.0 (2H,t), 4.3 (1H,m), 4.5 (1H,m), 4.9 (1H,m), 6.9 (1H,s), 7.0-7.4 (11H,m), 7.6 (1H,s), 7.7 (1H,t), 8.1 (1H,d), 8.3 (1H,d).
M.S. (m/z) (FAB) (M+1) = 770 (consistent with m.w. = 769.

Example 13a

2,3-Dihydro-3-oxo-4H-1,4-benzothiazine-2-acetyl-Phe-Leu-ACHPA 3-(1-imidazolyl)propylamide

This material was formed from 2,3-dihydro-3-oxo-4H-benzothiazine-2-acetic acid [7](0.11g), following the procedure of Example 8a. The crude product was chromatographed on silica gel using methanol/chloroform (0-8% methanol, gradient). This gave the title compound (0.28g).
NMR (δ) ($DMSOd_6$): 0.7-1.9 (24H,m), 2.1 (2H,m), 2.35 (1H,m), 2.6-2.85 (2H,m), 3.0 (3H,m), 3.75 (1H,m), 3.85 (2H,bs), 3.95 (2H,t), 4.3 (1H,m), 4.55 (1H,m), 4.8-4.9 (1H,2xd), 6.85 (1H,s), 7.0 (2H,m), 7.1-7.35 (9H,m), 7.6 (1H,s), 7.75 (1H,m), 8.05 (1H,m), 8.35 (1H,m), 10.6 (1H,s).
M.S. (m/z) (FAB) (M+1) = 788 (consistent with m.w. = 787).

Example 13b

2,3-Dihydro-3-oxo-4H-1,4-benzothiazine-2-acetyl-Phe-Leu-ACHPA 3-(1-imidazolyl)propylamide.HCl.$2H_2O$

This material was formed from the corresponding free base (Example 13a) (0.27g), following the procedure of Example 8b. This gave the title compound (0.27g) as a white powder.
NMR (δ) ($DMSOd_6$): 0.7-1.8 (22H,m), 1.9 (2H,m), 2.1 (2H,m), 2.35 (1H,m), 2.6-3.2 (5H,m), 3.75 (1H,m), 3.85 (2H,bs), 4.2 (2H,t), 4.3 (1H,m), 4.5 (1H,m), 7.0 (2H,m), 7.1-7.4 (8H,m), 7.7 (1H,s), 7.8 (1H,s), 7.85 (1H,m), 8.1 (1H,m), 8.3-8.5 (1H,m), 9.1 (1H,s), 10.6 (1H,s), 14.5 (1H,b).
Analysis: $C_{42}H_{57}N_7O_6S$.HCl.$2H_2O$ requires C,58.6; H,7.3; N,11.4%. Found: C,58.5; H,6.9; N,11.3%.

Example 14

6-Benzyloxycarbonyl-2,3-dihydro-3-oxo-4H-1,4-benzoxazine-4-propanoyl-Phe-Leu-ACHPA 3-(1-imidazolyl)-propylamide

This material was formed from 6-benzyloxycarbonyl-2,3-dihydro-3-oxo-4H-1,4-benzoxazine-4-propanoic acid (Description 13(f)) (0.20g) following the procedure of Example 8a. The crude product was purified by chromatography on silica gel using methanol/chloroform (0-5% methanol, gradient) to give the title compound (0.26g) as a white solid.
NMR (δ) ($DMSOd_6$): 0.6-1.8 (24H,m), 1.9 (2H,t), 2.1 (2H,d), 2.4 (2H,m), 2.7 (1H,m), 3.0 (3H,m), 3.7-4.7 (13H,m), 5.5 (2H,s), 7.0-7.5 (13H,m), 7.6 (2H,m), 7.7 (1H,m
M.S. (m/z) (FAB) (M+1) = 920 (consistent with m.w. = 919.

Example 15a

6-Carboxy-2,3-dihydro-3-oxo-4H-1,4-benzoxazine-4-propanoyl-Phe-Leu-ACHPA 3-(1-imidazolyl)propylamide

This material was formed from 6-benzyloxycarbonyl-2,3-dihydro-3-oxo-4H-1,4-benzoxazine-4-propanoyl-Phe-Leu-ACHPA 3-(1-imidazolyl)propylamide (Example 14) (0.25g), following the procedure of Description

[7] Ind. J. Chem. Vol.22B, 868 (1983).

1(b). Removal of solvent gave the title compound (0.20g) as a white solid.
NMR (δ) ($DMSOd_6$): 0.7-1.9 (24H,m), 2.1 (2H,m), 2.4-2.6 (3H,m), 2.6-2.8 (1H,m), 2.9-3.1 (4H,m), 3.8-4.0 (3H,m), 4.0-4.1 (3H,m), 4.3 (1H,m), 4.6 (1H,m), 4.7 (2H,s), 4.9 (1H,bs), 7.1 (2H,m), 7.1-7.4 (7H,m), 7.6 (2H,m), 7.8 (1H,m), 7.9 (1H,s), 8.2 (1H,d), 8.3 (1H,m).
M.S. (m/z) (FAB) (M+1) = 830 (consistent with m.w. = 829).

Example 15b

6-Carboxy-2,3-dihydro-3-oxo-4H-1,4-benzoxazine-4-propanoyl-Phe-Leu-ACHPA 3-(1-imidazolyl)propylamide .HCl.2.5$H_2O$

This material was formed from the corresponding free base (Example 15a) (0.19g), following the procedure of Example 8b to give the title compound (0.17g) as a white solid.
NMR (δ) ($DMSOd_6$): 0.6-1.8 (25H,m), 1.9 (2H,t), 2.1 (2H,m), 2.4 (2H,m), 2.7 (1H,m), 3.0 (3H,m), 3.8-4.7 (13H,m), 7.0-7.9 (13H,m), 8.2 (1H,d), 8.3 (1H,d), 9.1 (1H,s), 14.6 (1H,bs).
Analysis: $C_{44}H_{59}N_7O_9$.HCl.2.5$H_2O$ requires C,58.0; H,7.2; N,10.8%. Found: C,57.6; H,6.7; N,10.5%.

Example 16

6-(Benzyloxycarbonylmethyl-2,3-dihydro-3-oxo-4H-1,4-benzoxazine-4-propanoyl-Phe-Leu-ACHPA 3-(1-imidazolyl)propylamide

This material was formed from 6-(benzyloxycarbonylmethyl)-2,3-dihydro-3-oxo-4H-1,4-benzoxazine-4-propanoic acid (Description 14(f)) (0.11g), following the procedure of Example 8a. The title compound (0.13g) was isolated as a white solid.
NMR (δ) ($DMSOd_6$): 0.6-1.9 (24H,m), 1.9 (2H,t), 2.1 (2H,m), 2.4 (2H,m), 2.8 (1H,m), 3.1 (3H,m), 3.5 (2H,s), 3.7-3.9 (3H,m), 4.0 (1H,m), 4.2 (2H,m), 4.3 (1H,m), 4.5 (3H,m), 5.5 (2H,s), 7.0-7.6 (15H,m), 7.7 (1H,m), 7.9 (1H,s), 8.2 (1H,d), 8.3-8.4 (2H,m).
M.S. (m/z) (FAB) (M+1) 934 (consistent with m.w. = 933).

Example 17a

6-(Carboxymethyl)-2,3-dihydro-3-oxo-4H-1,4-benzoxazine-4-propanoyl-Phe-Leu-ACHPA 3-(1-imidazolyl)-propylamide

This material was formed from 6-(benzyloxycarbonylmethyl)-2,3-dihydro-3-oxo-4H-1,4-benzoxazine-4-propanoyl-Phe-Leu-ACHPA 3-(1-imidazolyl)propylamide (Example 16) (0.12g), following the procedure of Description 1(b). The title compound (0.090g) was isolated as a brown solid.
NMR (δ) ($DMSOd_6$): 0.8-1.9 (24H,m), 2.1 (2H,m), 2.3-2.4 (2H,m), 2.7-2.8 (1H,m), 2.9-3.1 (4H,m), 3.5 (2H,m), 3.7-3.9 (3H,m), 3.9-4.0 (3H,m), 4.0-4.3 (3H,m), 4.5 (3H,m), 6.8-6.9 (3H,m), 7.0 (1H,s), 7.1-7.4 (6H,m), 7.6 (1H,m), 7.7 (1H,m), 8.1-8.4 (2H,m).
M.S. (m/z) (FAB) (M+1) = 844 (consistent with m.w. = 843).

Example 17b

6-(Carboxvmethyl)-2,3-dihydro-3-oxo-4H-1,4-benzoxazine-4-propanoyl-Phe-Leu-ACHPA 3-(1-imidazolyl)-propylamide .HCl

This material was formed from the corresponding free base (Example 17a) (0.085g), following the procedure of Example 8(b). The title compound (0.086g) was isolated as a light brown solid.

NMR (δ) ($DMSOd_6$): 0.6-1.8 (24H,m), 1.9 (2H,m), 2.1 (2H,m), 2.4 (2H,m), 2.7 (1H,m), 3.5 (2H,s), 3.7-3.9 (3H,m), 4.0 (1H,m), 4.2 (2H,t), 4.3 (1H,m), 4.5 (3H,m), 6.9-7.5 (13H,m), 7.7 (1H,s), 7.8 (1H,s), 7.9 (1H,m), 8.3 (1H,m), 8.4 (1H,m), 14.4 (1H,bs).

Example 18

3,4-Dihydro-4-oxo-1,5-benzothiazepine-5(2H)-propanoyl-Phe-Leu-ACHPA 3-(1-imidazolyl)propylamide.0.5-($CHCl_3$)

This material was formed from 3,4-dihydro-4-oxo-1,5-benzothiazepine-5(2H)-propanoic acid (Description 15(b)) (0.12g) following the procedure of Example 8a. The crude product was purified by chromatography on silica gel using methanol/chloroform (0-10% methanol, gradient) to give the title compound (0.18g) as a white solid.
NMR (δ) ($DMSOd_6$): 0.6-1.9 (27H,m), 2.0-2.2 (2H,m), 2.2-2.6 (3H,m), 2.6-3.1 (4H,m), 3.1-3.6 (2H,m), 3.8-3.9 (2H,m), 3.9-4.0 (2H,m), 4.0-4.3 (4H,m), 4.4-4.5 (1H,m), 4.8-4.9 (1H,m), 6.9 (1H,s), 7.4-7.5 (2H,m), 7.6 (2H,m), 7.7 (1H,m), 8.1 (1H,d), 8.2 (1H,m), 8.3 (1H,s).
Analysis: $C_{44}H_{61}N_7O_6S.0.5(CHCl_3)$ requires C,61.0; H,7.1; N,11.2%. Found: C,60.7; H,7.1; N,11.1%.
M.S. (m/z) (FAB) (M+1) = 816 (consistent with m.w. = 815).

Example 19a

3,4-Dihydro-1,1,4-trioxo-1,5-benzothiazepine-5(2H)-propanoyl-Phe-Leu-ACHPA 3-(1-imidazolyl)-propylamide.2.5$H_2O$

This material was formed from 3,4-dihydro-1,1,4-trioxo-1,5-benzothiazepine-5(2H)-propanoic acid (Description 16(b)) (0.15g), following the procedure of Example 8a. The crude product was purified by chromatography on silica gel using methanol/chloroform (0-10% methanol, gradient) to give the title compound (0.11g) as a slightly coloured solid.
NMR (δ) ($DMSOd_6$): 0.6-1.9(27H,m), 2.0-2.2(2H,m), 2.4-2.6(1H,m), 2.6-2.8(1H,m), 2.8-3.2(3H,m), 3.6-4.0-(6H,m), 4.2-4.4(1H,m), 4.4-4.5(1H,m), 4.8-4.9(1H,m), 6.9(1H,s), 7.1-7.3(8H,m), 7.5-7.9(5H,m), 7.9(1H,dd), 8.1-(1H,d), 8.2(1H,m).
Analysis: $C_{44}H_{61}N_7O_8S.2.5H_2O$ requires C,59.2; H,7.4; N,11.0%. Found: C,59.4; H,7.0; N,10.7%.
M.S. (m/z) (FAB) (M+1)=848 (consistent with m.w.=847).

Example 19b

3,4-Dihydro-1,1,4-trioxo-1,6-benzothiazepine-5(2H)-propanoyl-Phe-Leu-ACHPA 3-(1-imidazolyl)-propylamide.HCl.1.5$H_2O$

This material was formed from the corresponding free base (Example 19a) (0.10g), following the procedure of Example 8(b). This gave the title compound (0.09g) as a slightly coloured solid.
NMR (δ) ($DMSOd_6$): 0.6-1.8(27H,m), 1.9(2H,m), 2.1(2H,m), 2.7(1H,m), 2.8-3.1(4H,m), 3.5-3.9(4H,m), 3.9-4.6-(10H,m), 7.1-7.3(7H,m), 7.6(1H,t), 7.7(2H,m), 7.8-8.0(3H,m), 8.1-8.4(2H,m), 9.1(1H,s), 14.5(1H,bs).
Analysis: $C_{44}H_{61}N_7O_8S.HCl.1.5H_2O$ requires C,58.0; H,7.2; N,10.8%. Found: C,57.9; H,6.9; N,10.5%.

Example 20

6-(BOC-aminomethyl)-2,3-dihydro-3-oxo-4H-1,4-benzothiazine-4-propanoyl-Phe-Leu-ACHPA 3-(1-imidazolyl)-propylamide

This material was formed from 6-(BOC-aminomethyl)-2,3-dihydro-3-oxo-4H-1,4-benzothiazine-4-propanoic acid (Description 17(e)) (0.20g), following the procedure of Example 8(a). The crude product was chromatographed on silica gel using methanol/chloroform (0-8%) methanol, gradient). This gave the title compound (0.17g) as a white powder.
NMR (δ) ($CDCl_3$): 0.7-1.0(8H,m), 1.0-1.85(23H,m), 2.0(2H,m), 2.3-2.7(4H,m), 2.95-3.7(8H,m), 4.05(4H,m), 4.2-4.5(6H,m), 5.6(1H,bt), 6.8-7.1(4H,m), 7.1-7.8(m).
M.S. (m/z) (FAB) (M+1)=931 (consistent with m.w.=930).

Example 21

6-(Aminomethyl)-2,3-dihydro-3-oxo-4H-1,4-benzothiazine-4-propanoyl-Phe-Leu-ACHPA 3-(1-imidazolyl)-propylamide .$2CF_3CO_2H$

This material was formed from 6-(BOC-aminomethyl)-2,3-dihydro-3-oxo-4H-1,4-benzothiazine-4-propanoyl-Phe-Leu-ACHPA 3-(1-imidazolyl)propylamide (Example 20) (0.15g), following the procedure of Example 11. This gave the title compound (0.14g) as a fluffy white solid.
M.S. (m/z) (FAB) (M+1)=831 (consistent with m.w. of free base=830).

Example 22

6-(Aminomethyl)-2,3-dihydro-1,1,3-trioxo-4H-1,4-benzothiazine-4-propanoyl-Phe-Leu-ACHPA 3-(1-imidazolyl)Propylamide .$2CF_3CO_2H$

This material was formed from the compound of Description 17(d), by successive S-oxidation (Description 8(a)), hydrolysis (Description 1(c)), peptide coupling (Example 8 a ) and N-deprotection (Example 11).

Biological Data

In vitro human renin inhibition

Renin inhibitory activity was estimated as the percentage change in renin activity in human plasma in the presence and absence of compound. The source of plasma was blood taken from healthy volunteers. Renin activity was defined by the difference in angiotensin I levels between two halves of a sample, one incubated at 37° and the other at 4° for 2 h. Angiotensin I levels were measured using a $^{125}$I- angiotensin I radioimmunoassay kit (NEN/DuPont, Stevenage). Results were calculated as the mean of at least two, duplicate determinations and $IC_{50}$ values were calculated by linear regression analysis of at least three concentrations of compound.

The results were as follows:

| Compound | $IC_{50}$ (nm) |
|---|---|
| E1 | 47 |
| E2 | 220 |
| E7 | 13 |
| E11 | 25 |
| E15 | 42 |
| E18 | 8 |
| E19 | 22 |

## Claims

1. A compound of formula (I), or a pharmaceutically acceptable salt thereof:

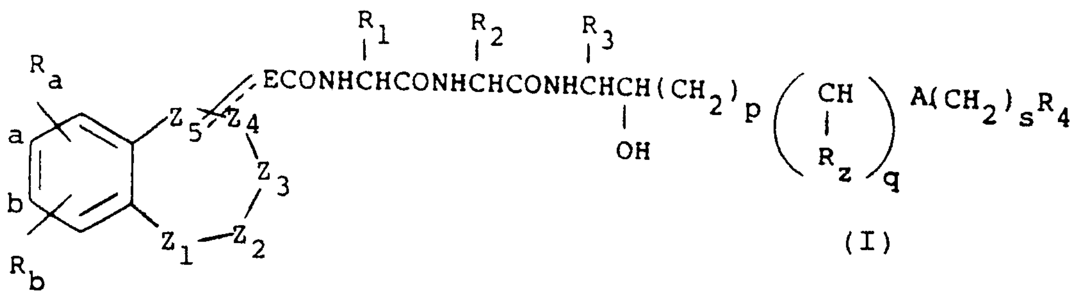

(I)

wherein
either $Z_1$ is absent and $Z_2$, $Z_3$, $Z_4$ and $Z_5$ and the carbon atoms to which $Z_2$ and $Z_5$ are attached, form a 6-membered non-aromatic heterocyclic ring; or
$Z_1$, $Z_2$, $Z_3$, $Z_4$, $Z_5$ and the carbon atoms to which $Z_1$ and $Z_5$ are attached, form a 7-membered non-aromatic heterocyclic ring;
E is absent or is $(CH_2)n$ or $CH(CH_2)_{n-1}$ wherein n is 1 to 4;
A is -CONH-, -NHCO-, -COO-, $-S(O)_r-$ wherein r is 0, 1 or 2, or $-CH_2-$;
p is 0, 1 or 2;
s is 0, 1, 2, 3 or 4;
q is 0 or 1;
$R_z$ is hydrogen, $C_{1-6}$ alkyl or, when A is $-CH_2-$, hydroxy;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl, $C_{3-8}$ cycloalkyl or phenyl;
$R_4$ is $C_{1-6}$ alkyl, $C_{3-8}$ cycloalkyl, a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and
the dashed line represents an optional bond (when E is present).

2. A compound according to claim 1 wherein $Z_2$ is $SO_2$ or O, $Z_3$ is $CH_2$, $Z_4$ is $CH_2$, $Z_5$ is CH and E is attached at $Z_5$; or $Z_2$ is $SO_2$, $Z_3$ is CH, $Z_4$ is CO, and $Z_5$ is N and E is attached at $Z_5$.

3. A compound according to claim 1 wherein $Z_1$ is present and $Z_1$, $Z_2$, $Z_3$, $Z_4$ and $Z_5$ are selected from:

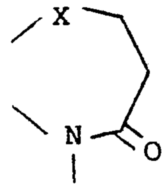 , 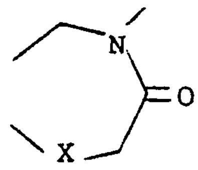 , 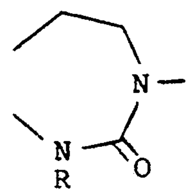 , 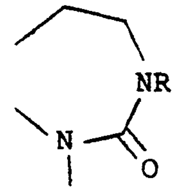

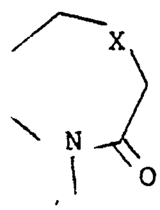 , 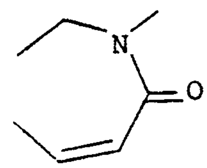 , 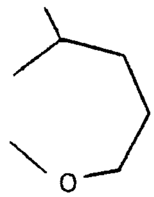

wherein X is O, S, $SO_2$, NH, N-$C_{1-6}$alkyl or $CH_2$.

4. A compound according to any one of claims 1 to 3 wherein one of $R_a$ and $R_b$ is hydrogen and the other is $CH_2NH_2$, $CO_2H$, $CH_2NHCH_2CO_2H$ or $SO_2CH_2CO_2H$.

5. A compound according to any one of claims 1 to 4 wherein the amino acid residue containing $R_2$ is Leu, $\beta$-pyrazolylalanine or His.

6. A compound according to claim 1 of formula (IA) or a pharmaceutically acceptable salt thereof:

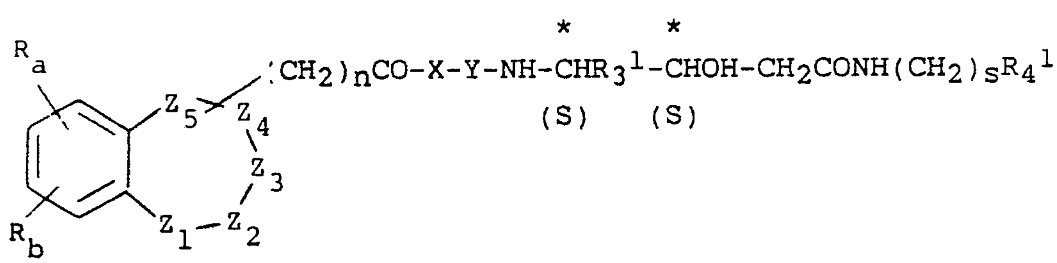

wherein

X is Phe;

Y is Leu or His;

$R_3{}^1$ is cyclohexylmethyl;

$R_4{}^1$ is $C_{4-5}$ alkyl and s is 0 or s is 2 to 4 and $R_4{}^1$ is carboxy or a saturated or unsaturated heterocyclic ring; and

the remaining variables are as defined in claim 1.

7. A compound according to any one of claims 1 to 6 wherein E is $(CH_2)_n$ wherein n is 1, 2 or 3 and E is attached at $Z_4$ or $Z_5$.

8. A compound according to any one of claims 1 to 7 wherein the sequence from the amino acid containing $R_3$ or $R_3{}^1$ to $NH(CH_2)_sR_4$, is 4(S)amino-5-cyclohexyl-3(S)-hydroxypentanoic acid (ACHPA).

9. A compound according to any one of claims 1 to 8 wherein s is 0 and $R_4$ is isobutyl or s is 2, 3 or 4 and $R_4$ is 1-imidazolyl, 2-imidazolyl, 4-morpholinyl, 2-oxopyrrolidin-1-yl, 2-pyridyl, 2-pyridyl-N-oxide, 3-pyridyl, hydroxyethyl, acetylamino, 4-methylpiperazin-1-yl, piperazin-1-yl or dimethylamino.

10. A compound selected from the group consisting of:

chromanyl-4-acetyl-Phe-Leu-ACHPA isobutylamide,

2,3-dihydro-6-methyl-3-oxo-4 H -1,4-benzoxazine-4-acetyl-Phe-Leu-ACHPA isobutylamide,

1,1-dioxothiachromanylidene-4-acetyl-Phe-Leu-ACHPA isobutylamide,

1,1-dioxothiachromanyl-4-acetyl-Phe-Leu-ACHPA isobutylamide,
6-cyano-2,3-dihydro-3-oxo-4H-1,4-benzoxazine-4-propanoyl-Phe-Leu-ACHPA isobutylamide,
2,3-dihydro-3-oxo-4H-1,4-benzothiazine-4-propanoyl-Phe-Leu-ACHPA isobutylamide,
2,3-dihydro-1,1,3-trioxo-4H-1,4-benzothiazine-4-propanoyl-Phe-Leu-ACHPA isobutylamide,
2,3-dihydro-1,1,3-trioxo-4H-1,4-benzothiazine-4-propanoyl-Phe-Leu-ACHPA 3-(1-imidazolyl)propylamide,
2,3-dioxo-1,2,3,4-tetrahydroquinoxaline-1-acetyl-Phe-Leu-ACHPA isobutylamide,
6-(BOC-aminomethyl)-2,3-dihydro-3-oxo-4H-1,4-benzoxazine-4-propanoyl-Phe-Leu-ACHPA 3-(1-imidazolyl)propylamide,
6-(aminomethyl)-2,3-dihydro-3-oxo-4H-1,4-benzoxazine-4-propanoyl-Phe-Leu-ACHPA 3-(1-imidazolyl)-propylamide,
3-(1,2,3,4-tetrahydroisoquinolin-2-yl)propanoyl-Phe-Leu-ACHPA 3-(1-imidazolyl)propylamide,
2,3-dihydro-3-oxo-4H-1,4-benzothiazine-2-acetyl-Phe-Leu-ACHPA 3-(1-imidazolyl)propylamide,
6-benzyloxycarbonyl-2,3-dihydro-3-oxo-4H-1,4-benzoxazine-4-propanoyl-Phe-Leu-ACHPA 3-(1-imidazolyl)-propylamide,
6-carboxy-2,3-dihydro-3-oxo-4H-1,4-benzoxazine-4-propanoyl-Phe-Leu-ACHPA 3-(1-imidazolyl)-propylamide,
6-(benzyloxycarbonylmethyl)-2,3-dihydro-3-oxo-4H-1,4-benzoxazine-4-propanoyl-Phe-Leu-ACHPA 3-(1-imidazolyl)propylamide,
6-(carboxymethyl)-2,3-dihydro-3-oxo-4H-1,4-benzoxazine-4-propanoyl-Phe-Leu-ACHPA 3-(1-imidazolyl)-propylamide,
3,4-dihydro-4-oxo-1,5-benzothiazepine-5(2H)-propanoyl-Phe-Leu-ACHPA 3-(1-imidazolyl)propylamide,
3,4-dihydro-1,1,4-trioxo-1,5-benzothiazepine-5(2H)-propanoyl-Phe-Leu-ACHPA 3-(1-imidazolyl)propylamide,
6-(BOC-aminomethyl)-2,3-dihydro-3-oxo-4H-1,4-benzo-thiazine-4-propanoyl-Phe-Leu-ACHPA 3-(1-imidazolyl)propylamide,
6-(aminomethyl)-2,3-dihydro-3-oxo-4H-1,4-benzothiazine-4-propanoyl-Phe-Leu-ACHPA 3-(1-imidazolyl)-propylamide and
6-(aminomethyl)-2,3-dihydro-1,1,3-trioxo-4H-1,4-benzothiazine-4-propanoyl-Phe-Leu-ACHPA 3-(1-imidazolyl)propylamide.

11. A process for the preparation of a compound according to claim 1, which process comprises reacting a reagent of the formula (III):

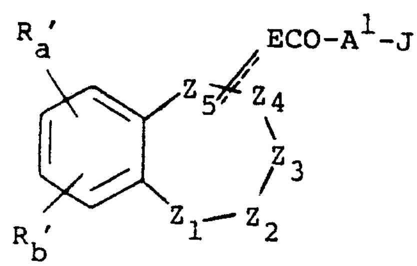

(III)

wherein $R_a'$ and $R_b'$ are $R_a$ and $R_b$ respectively or groups or atoms convertible thereto; $A^1$ is absent or represents an appropriate amino acid or dipeptide unit; J is OH or a leaving group; and the remaining variables are as defined in claim 1; with a reagent of the formula (IV):

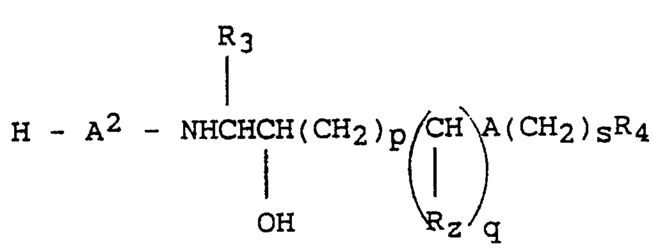

(IV)

wherein
$A^2$ is absent or represents an appropriate amino acid or dipeptide unit, such that $A^1$ + $A^2$ is

$-NHCHR_1CONHCHR_2CO-$, and the remaining variables are as defined in claim 1; and thereafter, if desired or necessary deprotecting (within $A^1$ or $A^2$) of the products, and/or converting $Z_1$, $Z_2$, $Z_3$, $Z_4$ or $Z_5$ to other $Z_1$, $Z_2$ $Z_3$, $Z_4$ or $Z_5$, $R_a'/R_b'$ to $R_a$ and/or $R_b$ and/or forming a pharmaceutically acceptable salt thereof.

12. A pharmaceutical composition comprising a compound according to any one of claims 1 to 10, and a pharmaceutically acceptable carrier.

13. A compound according to any one of claims 1 to 10 for use as an active therapeutic substance.

14. A compound according to any one of claims 1 to 10 for use in the treatment of hypertension.

15. Use of a compound according to any one of claims 1 to 10 in the manufacture of a medicament for use in the treatment of hypertension.

Claims for the following Contracting State: ES

1. A process for the preparation of a compound of formula (I), or a pharmaceutically acceptable salt thereof:

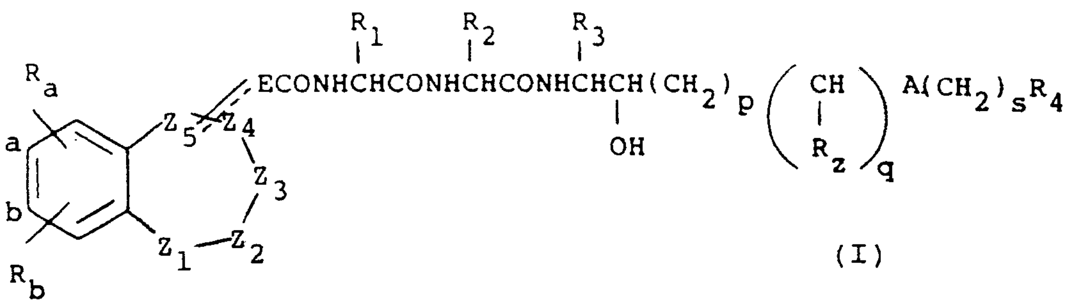

(I)

wherein

either $Z_1$ is absent and $Z_2$, $Z_3$, $Z_4$ and $Z_5$ and the carbon atoms to which $Z_2$ and $Z_5$ are attached, form a 6-membered non-aromatic heterocyclic ring; or

$Z_1$, $Z_2$, $Z_3$, $Z_4$, $Z_5$ and the carbon atoms to which $Z_1$ and $Z_5$ are attached, form a 7-membered non-aromatic heterocyclic ring;

E is absent or is $(CH_2)_n$ or $CH(CH_2)_{n-1}$ wherein n is 1 to 4;

A is -COHN-, -NHCO-, -COO-, $-S(O)_r$ wherein r is 0, 1 or 2, or $-CH_2-$;

p is 0, 1 or 2;

s is 0, 1, 2, 3 or 4;

q is 0 or 1;

$R_z$ is hydrogen, $C_{1-6}$ alkyl or, when A is $-CH_2-$, hydroxy;

$R_a$ and $R_b$ are independently selected from hydrogen or a substituent;

$R_1$ is $CH_2R_9$ wherein $R_9$ is optionally substituted aryl or heteroaryl;

$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;

$R_3$ is $CH_2R_{12}$ wherein $R_{12}$ is $C_{1-6}$ alkyl, $C_{3-8}$ cycloalkyl or phenyl;

$R_4$ is $C_{1-6}$ alkyl, $C_{3-8}$ cycloalkyl, a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and

the dashed line represents an optional bond (when E is present);

which process comprises reacting a reagent of the formula (III):

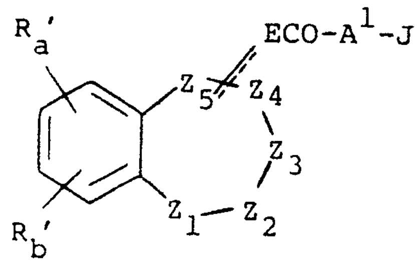

(III)

wherein $R_a'$ and $R_b'$ are $R_a$ and $R_b$ respectively or groups or atoms convertible thereto; $A^1$ is absent or represents an appropriate amino acid or dipeptide unit;J is OH or a leaving group; and the remaining variablesare as defined; with a reagent of the formula (IV):

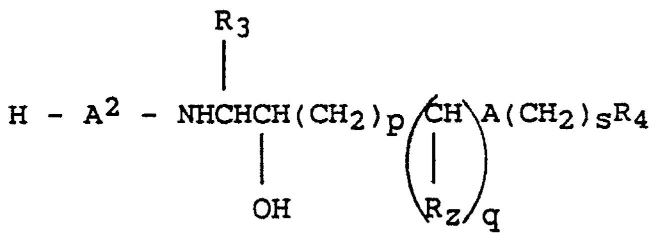

(IV)

wherein

$A^2$ is absent or represents an appropriate amino acid or dipeptide unit, such that $A^1$ + $A^2$ is $-NHCHR_1CONHCHR_2CO-$, and the remaining variables are as defined; and thereafter, if desired or necessary deprotecting (within $A^1$ or $A^2$) of the products, and/or converting $Z_1$, $Z_2$, $Z_3$, $Z_4$ or $Z_5$ to other $Z_1$, $Z_2$, $Z_3$, $Z_4$ or $Z_5$, $R_a'/R_b'$ to $R_a$ and/or $R_b$ and/or forming a pharmaceutically acceptable salt thereof.

2. A process according to claim 1 wherein $Z_2$ is $SO_2$ or O, $Z_3$ is $CH_2$, $Z_4$ is $CH_2$, $Z_5$ is CH and E is attached at $Z_5$; or $Z_2$ is $SO_2$, $Z_3$ is CH, $Z_4$ is CO, and $Z_5$ is N and E is attached at $Z_5$.

3. A process according to claim 1 wherein $Z_1$ is present and $Z_1$, $Z_2$, $Z_3$, $Z_4$ and $Z_5$ are selected from:

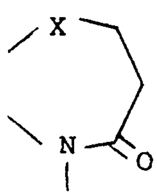 , 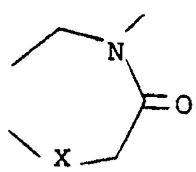 , 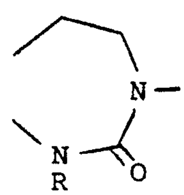 , 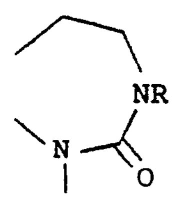

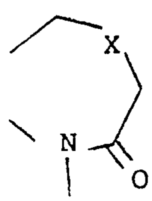 , 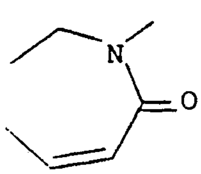 , 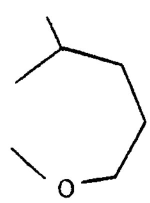

wherein X is O, S, $SO_2$, NH, N-$C_{1-6}$alkyl or $CH_2$.

4. A process according to any one of claims 1 to 3 wherein one of $R_a$ and $R_b$ is hydrogen and the other is

$CH_2NH_2$, $CO_2H$, $CH_2NHCH_2CO_2H$ or $SO_2CH_2CO_2H$.

5. A process according to any one of claims 1 to 4 wherein the amino acid residue containing $R_2$ is Leu, β-pyrazolylalanine or His.

6. A process according to claim 1 for the preparation of a compound of formula (IA) or a pharmaceutically acceptable salt thereof:

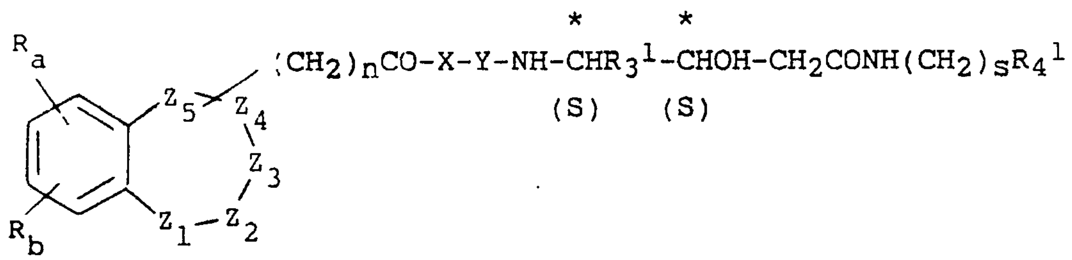

wherein

X is Phe;

Y is Leu or His;

$R_3{}^1$ is cyclohexylmethyl;

$R_4{}^1$ is $C_{4-5}$ alkyl and s is 0 or s is 2 to 4 and $R_4{}^1$ is carboxy or a saturated or unsaturated heterocyclic ring; and

the remaining variables are as defined in claim 1.

7. A process according to any one of claims 1 to 6 wherein E is $(CH_2)_n$ wherein n is 1, 2 or 3 and E is attached at $Z_4$ or $Z_5$.

8. A process according to any one of claims 1 to 7 wherein the sequence from the amino acid containing $R_3$ or $R_3{}^1$ to $NH(CH_2)_sR_4$, is 4(S)-amino-5-cyclohexyl-3(S)-hydroxypentanoic acid (ACHPA).

9. A process according to any one of claims 1 to 8 wherein s is 0 and $R_4$ is isobutyl or s is 2, 3 or 4 and $R_4$ is 1-imidazolyl, 2-imidazolyl, 4-morpholinyl, 2-oxopyrrolidin-1-yl, 2-pyridyl, 2-pyridyl-N-oxide, 3-pyridyl, hydroxyethyl, acetylamino, 4-methylpiperazin-1-yl, piperazin-1-yl or dimethylamino.

10. A process according to claim 1 for the preparation of compound selected from the group consisting of:
chromanyl-4-acetyl-Phe-Leu-ACHPA isobutylamide,
2,3-dihydro-6-methyl-3-oxo-4 H -1,4-benzoxazine-4-acetyl-Phe-Leu-ACHPA isobutylamide,
1,1-dioxothiachromanylidene-4-acetyl-Phe-Leu-ACHPA isobutylamide,
1,1-dioxothiachromanyl-4-acetyl-Phe-Leu-ACHPA isobutylamide,
6-cyano-2,3-dihydro-3-oxo-4 H -1,4-benzoxazine-4-propanoyl-Phe-Leu-ACHPA isobutylamide,
2,3-dihydro-3-oxo-4 H -1,4-benzothiazine-4-propanoyl-Phe-Leu-ACHPA isobutylamide,
2,3-dihydro-1,1,3-trioxo-4 H -1,4-benzothiazine-4-propanoyl-Phe-Leu-ACHPA isobutylamide,
2,3-dihydro-1,1,3-trioxo-4 H -1,4-benzothiazine-4-propanoyl-Phe-Leu-ACHPA 3-(1-imidazolyl)propylamide,
2,3-dioxo-1,2,3,4-tetrahydroquinoxaline-1-acetyl-Phe-Leu-ACHPA isobutylamide,
6-(BOC-aminomethyl)-2,3-dihydro-3-oxo-4 H -1,4-benzoxazine-4-propanoyl-Phe-Leu-ACHPA 3-(1-imidazolyl)propylamide,
6-(aminomethyl)-2,3-dihydro-3-oxo-4 H -1,4-benzoxazine-4-propanoyl-Phe-Leu-ACHPA 3-(1-imidazolyl)-propylamide,
3-(1,2,3,4-tetrahydroisoquinolin-2-yl)propanoyl-Phe-Leu-ACHPA 3-(1-imidazolyl)propylamide,
2,3-dihydro-3-oxo-4 H -1,4-benzothiazine-2-acetyl-Phe-Leu-ACHPA 3-(1-imidazolyl)propylamide,
6-benzyloxycarbonyl-2,3-dihydro-3-oxo-4 H -1,4-benzoxazine-4-propanoyl-Phe-Leu-ACHPA 3-(1-imidazolyl)-propylamide,
6-carboxy-2,3-dihydro-3-oxo-4 H -1,4-benzoxazine-4-propanoyl-Phe-Leu-ACHPA 3-(1-imidazolyl)-propylamide,
6-(benzyloxycarbonylmethyl)-2,3-dihydro-3-oxo-4 H -1,4-benzoxazine-4-propanoyl-Phe-Leu-ACHPA 3-(1-imidazolyl)propylamide,
6-(carboxymethyl)-2,3-dihydro-3-oxo-4 H -1,4-benzoxazine-4-propanoyl-Phe-Leu-ACHPA 3-(1-imidazolyl)-propylamide,
3,4-dihydro-4-oxo-1,5-benzothiazepine-5(2 H )-propanoyl-Phe-Leu-ACHPA 3-(1-imidazolyl)propylamide.
3,4-dihydro-1,1,4-trioxo-1,5-benzothiazepine-5(2 H )-propanoyl-Phe-Leu-ACHPA 3-(1-imidazolyl)propylamide,
6-(BOC-aminomethyl)-2,3-dihydro-3-oxo-4 H -1,4-benzo-thiazine-4-propanoyl-Phe-Leu-ACHPA 3-(1-imidazolyl)propylamide,

6-(aminomethyl)-2,3-dihydro-3-oxo-4 H -1,4-benzothiazine-4-propanoyl-Phe-Leu-ACHPA 3-(1-imidazolyl)-propylamide and
6-(aminomethyl)-2,3-dihydro-1,1,3-trioxo-4 H -1,4-benzothiazine-4-propanoyl-Phe-Leu-ACHPA 3-(1-imidazolyl)propylamide.

11. Use of a compound of formula (I) as defined in any one of claims 1 to 10 or a pharmaceutically acceptable salt thereof, in the manufacture of a medicament for use in the treatment of hypertension.

European Patent Office

# EUROPEAN SEARCH REPORT

Application Number

EP 90 30 5978

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | WO-A-8 803 022 (PFIZER)<br>* Page 3 - page 9, summary *<br>--- | 1 | C 07 K 5/02<br>A 61 K 37/64 |
| Y | PEPTIDES: PROCEEDINGS OF THE TENTH AMERICAN PEPTIDE SYMPOSIUM, St. Louis, Missouri, 23rd - 28th May 1987, ed. G.R. MARSHALL, 1987, pages 474-475, ESCOM, Leiden, NL; D.J. KEMPF et al.: "Renin inhibitors based on novel dipeptide analogs: Increased efficacy through systematic inclusion of polar functionality"<br>* Whole document *<br>----- | 1 | |

TECHNICAL FIELDS SEARCHED (Int. Cl.5)

C 07 K
A 61 K

The present search report has been drawn up for all claims

| Place of search | Date of completion of the search | Examiner |
|---|---|---|
| THE HAGUE | 17-09-1990 | KORSNER S.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)